# EUROPEAN PATENT APPLICATION

(11) **EP 4 427 781 A1**
(43) Date of publication of application: **11.09.2024**
(21) Application number: 24161297.7
(22) Date of filing: 05.03.2024
(51) Int. Cl.: A61M 16/00

(54) **ANESTHESIA MACHINE, RESPIRATORY SUPPORT DEVICE**

(30) Priority: 05.03.2023 CN 202310239156
(71) Applicant: Shenzhen Mindray Bio-Medical Electronics Co., Ltd., Shenzhen, Guangdong 518057 (CN)
(72) Inventor: CAI, Kun, Shenzhen, 518057 (CN); ZHOU, Xiaoyong, Shenzhen, 518057 (CN); YANG, Shuwang, Shenzhen, 518057 (CN); CAO, Tianyuan, Shenzhen, 518057 (CN)
(74) Representative: KIPA AB

(57) **Abstract**

An anesthesia machine, a respiratory support device, and a ventilation control method are provided by this disclosure. The ventilation module provides a first gas at a first ventilation frequency and a second ventilation frequency, respectively. When the ventilation module provides the first gas to a patient at the second ventilation frequency, the ventilation module can simultaneously provide the first gas to the patient at the first ventilation frequency in at least one of the multiple second inspiratory phases. In this way, this disclosure provides respiratory support for a patient by superposing ventilation modes of two different frequencies, thereby improving an elimination rate of carbon dioxide, when providing the respiratory support for the patient.

## Description

### CROSS-REFERENCE TO RELATED APPLICATION

This disclosure claims priority to Chinese patent Application No. 202310239156.4, filed on March 5, 2023, the content of which is incorporated herein by reference in its entirety.

### TECHNICAL FIELD

The disclosure relates to a technical field of medical device, and more particularly to an anesthesia machine, a respiratory support device, and a ventilation control method.

### BACKGROUND

In an implementation process of anesthesia, unlike other surgeries, surgeries performed in a central airway pose challenges for anaesthesiologists and otolaryngologists, because they share the same airway. In this scenario, a surgical operation, and an observation of the otolaryngologists conflict with a ventilation task of the anaesthesiologist, making a use of conventional tracheal intubation impractical. Jet ventilation is a good solution. In order to reduce a movement of the surgical site caused by a gas flow, high-frequency jet ventilation (HFJV) has been developed and applied. HFJV adopts a higher ventilation frequency and lower tidal volume, which can maintain sufficient gas exchange. Although HFJV provides sufficient carbon dioxide elimination in most patients, for severe obstructive and/or restrictive lung disease patients, HFJV may not be able to fully remove carbon dioxide.

Therefore, existing ventilation modes still need to be improved and enhanced.

### SUMMARY

This disclosure mainly provides an anesthesia machine, a respiratory support device, and a ventilation control method, aiming to improve an elimination rate of carbon dioxide when providing respiratory support for a patient.

One embodiment provides an anesthesia machine, including an anesthesia branch and a ventilation module;
the anesthesia branch is configured to mix a first gas with an anesthetic, so as to obtain a second gas, and configured to provide the second gas to a patient, so as to provide anesthesia respiratory support for said patient;
the ventilation module is configured to respectively provide the first gas to a patient at a first ventilation frequency and a second ventilation frequency, so as to provide respiratory support for said patient; wherein the ventilation module is further configured to, when providing the first gas to said patient at the second ventilation frequency in multiple second inspiratory phases, simultaneously provide the first gas to said patient at the first ventilation frequency in at least one of the multiple second inspiratory phases.

One embodiment provides an anesthesia machine, including an anesthesia branch and a ventilation module;
the anesthesia branch is configured to mix a first gas with an anesthetic, so as to obtain a second gas, and configured to provide the second gas to a patient, so as to provide anesthesia respiratory support for said patient;
the ventilation module is configured to, when a respiratory support mode, which is provided by the anesthesia machine, is superimposed ventilation, simultaneously provide the first gas to a patient at a first ventilation frequency and a second ventilation frequency, so as to provide respiratory support for said patient.

In a preferable embodiment, the anesthesia machine further includes a human-machine interaction apparatus, which is configured to receive the respiratory support mode, which is provided by the anesthesia machine, wherein
the respiratory support mode is inputted by a user;
the ventilation module is further configured to:
   provide the first gas to said patient at the first ventilation frequency, so as to provide respiratory support for said patient, when the respiratory support mode, which is provided by the anesthesia machine, is high-frequency ventilation;
   provide the first gas to said patient at the second ventilation frequency, so as to provide respiratory support for said patient, when the respiratory support mode, which is provided by the anesthesia machine, is normal-frequency ventilation.

In a preferable embodiment, when the respiratory support mode, which is received by the human-machine interaction apparatus, is superimposed ventilation, the anesthesia machine is configured to:
display, on a display interface of the human-machine interaction apparatus, a first display control and a second display control;
receive, through the human-machine interaction apparatus, a user operation on the first display control, so as to obtain a first ventilation parameter; and receive, through the human-machine interaction apparatus, a user operation on the second first display control, so as to obtain a second ventilation parameter; wherein the first ventilation parameter includes at least one of a first ventilation frequency, a first inspiratory-to-expiratory ratio, and a first driving pressure, the second ventilation parameter includes at least one of a second ventilation frequency, a second inspiratory-to-expiratory ratio, and a second driving pressure; and
provide respiratory support for said patient according to the first ventilation parameter, and provide respiratory support for said patient according to the second ventilation parameter.

In a preferable embodiment, the anesthesia machine is further configured to acquire an airway pressure, and display, on the display interface, waveform(s) of the airway pressure.

In a preferable embodiment, the ventilation module includes a first ventilation apparatus and a second ventilation apparatus, which are capable of being controlled to work simultaneously;
the first ventilation apparatus is configured to provide the first gas to said patient at the first ventilation frequency;
the second ventilation apparatus is configured to provide the first gas to said patient at the second ventilation frequency.

In a preferable embodiment, the anesthesia machine further includes an oxygen interface, which is connected with an external oxygen source or an internal oxygen source, and/or an air interface, which is connected with an external air source or an internal air source; wherein the first gas includes oxygen and/or air;
the first ventilation apparatus and the second ventilation apparatus are both connected with the oxygen interface and/or the air interface.

In a preferable embodiment, the anesthesia machine further includes a fifth pressure regulation valve and a sixth pressure regulation valve, which are both configured to control a gas pressure; wherein the first ventilation apparatus is connected with the oxygen interface through the fifth pressure regulation valve, and/or connected with the air interface through the sixth pressure regulation valve; the second ventilation apparatus is connected with the oxygen interface through the fifth pressure regulation valve, and/or connected with the air interface through the sixth pressure regulation valve.

In a preferable embodiment, the first ventilation apparatus includes a first oxygen branch, a first air branch, and a first output port; an input end of the first oxygen branch is connected with the oxygen interface, and an input end of the first air branch is connected with the air interface; the first oxygen branch and the first air branch are converged with each other and then connected with the first output port; wherein the first oxygen branch is arranged with a first pressure regulation valve, which is configured to control an oxygen pressure inside the first oxygen branch; the first air branch is arranged with a second pressure regulation valve, which is configured to control an air pressure inside the first air branch; the second ventilation apparatus includes a second oxygen branch, a second air branch, and a second output port; an input end of the second oxygen branch is connected with the oxygen interface, and an input end of the second air branch is connected with the air interface; the second oxygen branch and the second air branch are converged with each other and then connected with the second output port; wherein the second oxygen branch is arranged with a third pressure regulation valve, which is configured to control an oxygen pressure inside the second oxygen branch; the second air branch is arranged with a fourth pressure regulation valve, which is configured to control an air pressure inside the second air branch; or
the anesthesia machine further includes a fifth pressure regulation valve and a sixth pressure regulation valve, which are both configured to control a gas pressure; the first ventilation apparatus includes a first oxygen branch, a first air branch, and a first output port; an input end of the first oxygen branch is connected with the oxygen interface through the fifth pressure regulation valve, and an input end of the first air branch is connected with the air interface through the sixth pressure regulation valve; the first oxygen branch and the first air branch are converged with each other and then connected with the first output port; the second ventilation apparatus includes a second oxygen branch, a second air branch, and a second output port; an input end of the second oxygen branch is connected with the oxygen interface through the fifth pressure regulation valve, and an input end of the second air branch is connected with the air interface through the sixth pressure regulation valve; the second oxygen branch and the second air branch are converged with each other and then connected with the second output port.

In a preferable embodiment, the first oxygen branch is arranged with a first control valve, which is located downstream of the first pressure regulation valve or the fifth pressure regulation valve; wherein the first control valve is configured to control a flow amount of oxygen inside the first oxygen branch and enable the oxygen inside the first oxygen branch to be provided to said patient at the first ventilation frequency; the first air branch is arranged with a second control valve, which is located downstream of the second pressure regulation valve or the sixth pressure regulation valve; wherein the second control valve is configured to control a flow amount of air inside the first air branch and enable the air inside the first air branch to be provided to said patient at the first ventilation frequency; the second oxygen branch is arranged with a third control valve, which is located downstream of the third pressure regulation valve or the fifth pressure regulation valve; wherein the third control valve is configured to control a flow amount of oxygen inside the second oxygen branch and enable the oxygen inside the second oxygen branch to be provided to said patient at the second ventilation frequency; the second air branch is arranged with a fourth control valve, which is located downstream of the fourth pressure regulation valve or the sixth pressure regulation valve; wherein the fourth control valve is configured to control a flow amount of air inside the second air branch and enable the air inside the second air branch to be provided to said patient at the second ventilation frequency; or
the first ventilation apparatus further includes a fifth control valve, which is located downstream of a converged node of the first oxygen branch and the first air branch; wherein the fifth control valve is configured to control a flow amount of the first gas which is formed by mixing air and oxygen and enable the first gas to be provided to said patient at the first ventilation frequency; the second ventilation apparatus further includes a sixth control valve, which is located downstream of a converged node of the second oxygen branch and the second air branch; wherein the sixth control valve is configured to control a flow amount of the first gas which is formed by mixing air and oxygen and enable the first gas to be provided to said patient at the second ventilation frequency.

In a preferable embodiment, the first ventilation apparatus includes a first oxygen branch and a first output port; an input end of the first oxygen branch is connected with the oxygen interface, and an output end of the first oxygen branch is connected with the first output port; wherein the first oxygen branch is arranged with a first pressure regulation valve, which is configured to control an oxygen pressure inside the first oxygen branch; the second ventilation apparatus includes a second oxygen branch and a second output port; an input end of the second oxygen branch is connected with the oxygen interface, and an output end of the second oxygen branch is connected with the second output port; wherein the second oxygen branch is arranged with a third pressure regulation valve, which is configured to control an oxygen pressure inside the second oxygen branch; or
the anesthesia machine further includes a fifth pressure regulation valve, which is configured to control a gas pressure; the first ventilation apparatus includes a first oxygen branch and a first output port; an input end of the first oxygen branch is connected with the oxygen interface through the fifth pressure regulation valve, and an output end of the first oxygen branch is connected with the first output port; the second ventilation apparatus includes a second oxygen branch and a second output port; an input end of the second oxygen branch is connected with the oxygen interface through the fifth pressure regulation valve, and an output end of the second oxygen branch is connected with the second output port.

In a preferable embodiment, the first oxygen branch is arranged with a first control valve, which is located downstream of the first pressure regulation valve or the fifth pressure regulation valve; wherein the first control valve is configured to control a flow amount of oxygen inside the first oxygen branch and enable the oxygen inside the first oxygen branch to be provided to said patient at the first ventilation frequency; and the second oxygen branch is arranged with a third control valve, which is located downstream of the third pressure regulation valve or the fifth pressure regulation valve; wherein the third control valve is configured to control a flow amount of oxygen inside the second oxygen branch and enable the oxygen inside the second oxygen branch to be provided to said patient at the second ventilation frequency.

In a preferable embodiment, the first ventilation apparatus includes a first air branch and a first output port; an input end of the first air branch is connected with the air interface, and an output end of the first air branch is connected with the first output port; wherein the first air branch is arranged with a second pressure regulation valve, which is configured to control an air pressure inside the first air branch; the second ventilation apparatus includes a second air branch and a second output port; an input end of the second air branch is connected with the air interface, and an output end of the second air branch is connected with the second output port; wherein the second air branch is arranged with a fourth pressure regulation valve, which is configured to control an air pressure inside the second air branch; or
the anesthesia machine further includes a sixth pressure regulation valve, which is configured to control a gas pressure; the first ventilation apparatus includes a first air branch and a first output port; an input end of the first air branch is connected with the air interface through the sixth pressure regulation valve, and an output end of the first air branch is connected with the first output port; the second ventilation apparatus includes a second air branch and a second output port; an input end of the second air branch is connected with the air interface through the sixth pressure regulation valve, and an output end of the second air branch is connected with the second output port.

In a preferable embodiment, the first air branch is arranged with a second control valve, which is located downstream of the second pressure regulation valve or the sixth pressure regulation valve; wherein the second control valve is configured to control a flow amount of air inside the first air branch and enable the air inside the first air branch to be provided to said patient at the first ventilation frequency;
the second air branch is arranged with a fourth control valve, which is located downstream of the fourth pressure regulation valve or the sixth pressure regulation valve; wherein the fourth control valve is configured to control a flow amount of air inside the second air branch and enable the air inside the second air branch to be provided to said patient at the second ventilation frequency.

In a preferable embodiment, a driving pressure of the first ventilation apparatus for providing respiratory support for said patient, is lower than a driving pressure of the second ventilation apparatus for providing respiratory support for said patient.

In a preferable embodiment, the first ventilation apparatus includes a first control component which is configured to control an oxygen concentration of the first gas, which is outputted by the first ventilation apparatus; the second ventilation apparatus includes a second control component which is configured to control an oxygen concentration of the first gas, which is outputted by the second ventilation apparatus; wherein the first control component and the second control component are independent control components.

In a preferable embodiment, the anesthesia machine further includes a first pressure regulation component, which is located upstream of the first control component, and a second pressure regulation component, which is located upstream of the second control component; wherein the first pressure regulation component and the second pressure regulation component are pressure regulation valves, which are configured to respectively regulate, through regulating opening degrees of the pressure regulation valves, a gas pressure which is provided by the first ventilation apparatus and a gas pressure which is provided by the second ventilation apparatus.

In a preferable embodiment, the first ventilation frequency is not lower than 50bpm, and the second ventilation frequency is lower than 100bpm.

In a preferable embodiment, in order to provide respiratory support for said patient, the anesthesia machine is further configured to provide different flow amounts of the first gas to said patient in two different time periods of a respiratory cycle; wherein a flow amount in one time period is lower than a flow amount in the other time period, or a flow amount in one time period is zero.

In a preferable embodiment, the anesthesia machine further includes a purging branch and an airway pressure sensor, wherein the airway pressure sensor is connected with a pressure sampling tube;
the purging branch is configured to provide a purging gas to the pressure sampling tube, and is arranged with a third control component; wherein the third control component is configured to start the purging branch, so as to enable the purging branch to provide the purging gas to the pressure sampling tube, when the ventilation module is started.

In a preferable embodiment, the anesthesia branch includes a monitoring apparatus for flow amount, an anesthetic delivery apparatus which is connected with the monitoring apparatus for flow amount, a respiratory loop, and a ventilation control apparatus; wherein:
the monitoring apparatus for flow amount is configured to regulate a flow amount of the first gas; the anesthetic delivery apparatus is configured to mix the first gas with the anesthetic, so as to obtain the second gas, and configured to provide the second gas to the respiratory loop; the respiratory loop is configured to provide the second gas to said patient; and the ventilation control apparatus is configured to control the respiratory loop to provide the second gas to said patient, so as to enable the anesthesia machine to provide anesthesia respiratory support for said patient.

In a preferable embodiment, the anesthesia machine further includes a housing, wherein at least part of the anesthesia branch and at least part of the ventilation module are arranged inside a containment space, which is formed by the housing.

One embodiment provides a respiratory support device, including a first ventilation apparatus and a second ventilation apparatus, which are capable of being controlled to work simultaneously, so as to provide respiratory support for a patient;
the first ventilation apparatus is configured to provide a first gas to the patient at a first ventilation frequency, and the second ventilation apparatus is configured to provide a first gas to the patient at a second ventilation frequency;
the first ventilation apparatus includes a first gas delivery branch and a first control component which is arranged inside the first gas delivery branch, wherein the first control component is configured to control an oxygen concentration of the first gas, which is outputted by the first ventilation apparatus through the first gas delivery branch; the second ventilation apparatus includes a second gas delivery branch and a second control component which is arranged inside the second gas delivery branch, wherein the second control component is configured to control an oxygen concentration of the first gas, which is outputted by the second ventilation apparatus through the second gas delivery branch; wherein the first control component and the second control component are independent control components.

In a preferable embodiment, the first gas delivery branch is further arranged with a first pressure regulation component, which is configured to control a pressure of a gas provided by the first ventilation apparatus; the second gas delivery branch is further arranged with a second pressure regulation component, which is configured to control a pressure of a gas provided by the second ventilation apparatus; wherein the first pressure regulation component is located upstream of the first control component, and the second pressure regulation component is located upstream of the second control component.

In a preferable embodiment, the first pressure regulation component and the second pressure regulation component are pressure regulation valves, which are configured to respectively regulate, through regulating opening degrees of the pressure regulation valves, the pressure of the gas provided by the first ventilation apparatus and the pressure of the gas provided by the second ventilation apparatus.

In a preferable embodiment, the respiratory support device further includes an oxygen interface, which is configured to provide oxygen; and/or an air interface, which is configured to provide air; wherein the first gas includes the oxygen and/or the air.

In a preferable embodiment, the respiratory support device further includes a fifth pressure regulation valve and a sixth pressure regulation valve, which are both configured to control a gas pressure;
wherein the first gas delivery branch is connected with the oxygen interface through the fifth pressure regulation valve, and/or connected with the air interface through the sixth pressure regulation valve; the second gas delivery branch is connected with the oxygen interface through the fifth pressure regulation valve, and/or connected with the air interface through the sixth pressure regulation valve; wherein the first control component and the second control component are respectively located downstream of the fifth pressure regulation valve and the sixth pressure regulation valve.

In a preferable embodiment, the first ventilation apparatus and the second ventilation apparatus are connected with the oxygen interface and/or the air interface through independent pipelines, so as to receive the first gas from the oxygen interface and the air interface, respectively.

In a preferable embodiment, the first ventilation apparatus further includes a first output port; the first gas delivery branch includes a first oxygen branch and a first air branch; an input end of the first oxygen branch is connected with the oxygen interface, an input end of the first air branch is connected with the air interface; the first oxygen branch and the first air branch are converged with each other and then connected with the first output port; wherein the first oxygen branch is arranged with a first pressure regulation valve, which is configured to control an oxygen pressure inside the first oxygen branch; the first air branch is arranged with a second pressure regulation valve, which is configured to control an air pressure inside the first air branch; the second ventilation apparatus further includes a second output port; the second gas delivery branch includes a second oxygen branch and a second air branch; an input end of the second oxygen branch is connected with the oxygen interface, and an input end of the second air branch is connected with the air interface; the second oxygen branch and the second air branch are converged with each other and then connected with the second output port; wherein the second oxygen branch is arranged with a third pressure regulation valve, which is configured to control an oxygen pressure inside the second oxygen branch; the second air branch is arranged with a fourth pressure regulation valve, which is configured to control an air pressure inside the second air branch; or
the respiratory support device further includes a fifth pressure regulation valve and a sixth pressure regulation valve, which are both configured to control a gas pressure; the first ventilation apparatus further includes a first output port; the first gas delivery branch includes a first oxygen branch and a first air branch; an input end of the first oxygen branch is connected with the oxygen interface through the fifth pressure regulation valve, and an input end of the first air branch is connected with the air interface through the sixth pressure regulation valve; the first oxygen branch and the first air branch are converged with each other and then connected with the first output port; the second ventilation apparatus further includes a second output port; the second gas delivery branch includes a second oxygen branch and a second air branch; an input end of the second oxygen branch is connected with the oxygen interface through the fifth pressure regulation valve, and an input end of the second air branch is connected with the air interface through the sixth pressure regulation valve; the second oxygen branch and the second air branch are converged with each other and then connected with the second output port.

One embodiment provides a ventilation control method for an anesthesia machine, including:
controlling a first gas to enter into a ventilation module of the anesthesia machine; and
controlling the ventilation module to provide the first gas to a patient at a first ventilation frequency and a second ventilation frequency, so as to provide respiratory support for the patient; wherein, when the ventilation module provides the first gas to the patient at the second ventilation frequency in multiple second inspiratory phases, controlling the ventilation module to simultaneously provide the first gas to the patient at the first ventilation frequency in at least one of the multiple second inspiratory phases.
In a preferable embodiment, the ventilation module includes a first ventilation apparatus and a second ventilation apparatus;
wherein, when the ventilation module provides the first gas to the patient at the second ventilation frequency in multiple second inspiratory phases, controlling the ventilation module to simultaneously provide the first gas to the patient at the first ventilation frequency in at least one second inspiratory phase of the multiple second inspiratory phases, includes:
   controlling the first ventilation apparatus to provide the first gas to the patient at the first ventilation frequency; and
   simultaneously controlling the second ventilation apparatus to provide the first gas to the patient at the second ventilation frequency;
   wherein the first ventilation frequency is higher than the second ventilation frequency.

In a preferable embodiment, the first ventilation apparatus includes a first gas delivery branch, the second ventilation apparatus includes a second gas delivery branch; the first gas delivery branch is arranged with a first pressure regulation component, wherein the first pressure regulation component is configured to control a pressure of a gas provided by the first ventilation apparatus; the second gas delivery branch is arranged with a second pressure regulation component, wherein the second pressure regulation component is configured to control a pressure of a gas provided by the second ventilation apparatus;
wherein the ventilation control method further includes:
displaying, on a display interface of a human-machine interaction apparatus of the anesthesia machine, a first display control and a second display control;
receiving, through the human-machine interaction apparatus, a user operation on the first display control, so as to obtain a first driving pressure, and receiving, through the human-machine interaction apparatus, a user operation on the second display control, so as to obtain a second driving pressure; and
controlling the first pressure regulation component according to the first driving pressure, so as to, when providing respiratory support for the patient, keep a driving pressure of the first ventilation apparatus to be consistent with the first driving pressure; and controlling the second pressure regulation component according to the second driving pressure, so as to, when providing respiratory support for the patient, keep a driving pressure of the second ventilation apparatus to be consistent with the second driving pressure.

According to the anesthesia machine, respiratory support device, and ventilation control method of above embodiments, the ventilation module can provide the first gas at the first ventilation frequency and the second ventilation frequency, respectively; and when providing the first gas to the patient at the second ventilation frequency in multiple second inspiratory phases, the ventilation module can simultaneously provide the first gas to the patient at the first ventilation frequency in at least one second inspiratory phase of the multiple second inspiratory phases. In this way, this disclosure provides respiratory support for a patient by superposing ventilation modes of two different frequencies, thereby improving an elimination rate of carbon dioxide, when providing the respiratory support for the patient.

### BRIEF DESCRIPTION OF THE DRAWINGS

FIG. 1 is a structural block diagram for an embodiment of an anesthesia system provided by this disclosure.
FIG. 2 is a structural block diagram for an embodiment of an anesthesia system provided by this disclosure.
FIG. 3 is a structural block diagram for an embodiment of an anesthesia system provided by this disclosure.
FIG. 4 is a waveform diagram of an airway pressure, which is generated by a ventilation module in an anesthesia system provided by this disclosure.
FIG. 5 is a gas path diagram of a high-frequency ventilation module and a normal-frequency ventilation module in existing ventilators.
FIG. 6 is a structural diagram of an embodiment of a ventilation module in an anesthesia system provided by this disclosure.
FIG. 7 is a gas path diagram of an embodiment of a ventilation module in an anesthesia system provided by this disclosure.
FIG. 8 is a gas path diagram of an embodiment of a ventilation module in an anesthesia system provided by this disclosure.
FIG. 9 is a gas path diagram of an embodiment of a ventilation module in an anesthesia system provided by this disclosure.
FIG. 10 is a gas path diagram of an embodiment of a ventilation module in an anesthesia system provided by this disclosure.
FIG. 11 is a gas path diagram of an embodiment of a ventilation module in an anesthesia system provided by this disclosure.
FIG. 12 is a gas path diagram of an embodiment of a ventilation module in an anesthesia system provided by this disclosure.
FIG. 13 is a structural block diagram for an embodiment of an anesthesia system provided by this disclosure.
FIG. 14 is a schematic diagram of a display interface in an anesthesia system provided by this disclosure.
FIG. 15 is a structural diagram which is related to airway pressure detection in an anesthesia system provided by this disclosure.
FIG. 16 is a structural block diagram of an embodiment of a respiratory support device provided by this disclosure.
FIG. 17 is a flowchart of an embodiment of a ventilation control method provided by this disclosure.
FIG. 18 is a flowchart for performing ventilation control in a ventilation control method provided by this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the specification are operable to distinguish different objects, rather than to describe a specific order. In addition, the terms "connection", and "linkage" mentioned in this disclosure include direct and indirect connection (linkage), unless otherwise specified.

This disclosure introduces a ventilation module, which provides respiratory support, into an anesthesia system, wherein the ventilation module is capable of providing for the patient respiratory support with two different ventilation frequencies at the same time. The advantage of the respiratory support at a high ventilation frequency, and the advantage of the respiratory support at a low ventilation frequency complement each other, which improves an elimination rate of carbon dioxide, when providing the respiratory support for patient. Some embodiments are provided below for detailed explanation.

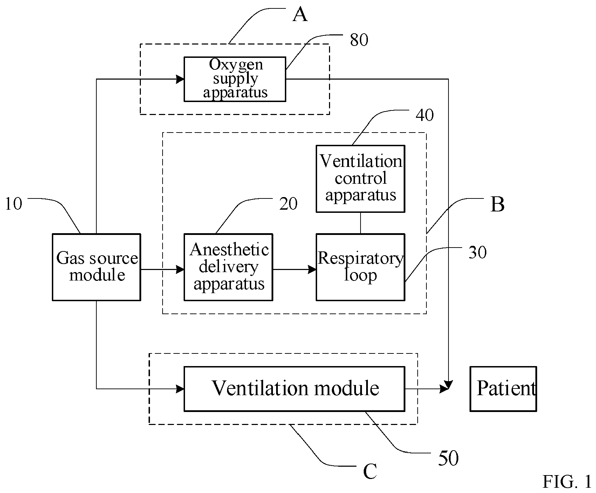

As shown in FIG. 1, an anesthesia system provided by this disclosure includes a gas source module 10, an oxygen supply branch A, an anesthesia branch B, and a ventilation branch C.

The gas source module 10 is configured to provide a gas, which is required by the anesthesia system, that is, to provide gas(es), which is(are) required by the oxygen supply branch A, the anesthesia branch B, and the ventilation branch C, such as to provide an oxygen-containing gas, which is required by the oxygen supply branch A, to provide a first gas, etc. The gas source module 10 can specifically provide one or more of oxygen, air, and laughing gas (nitrous oxide), as the anesthesia system needs to provide respiratory support for patient, accordingly the provided gas contains at least oxygen. That is, the first gas can be an oxygen-containing gas, such as oxygen; air; a mixture of oxygen and air; a mixture of oxygen and laughing gas; or a mixture of oxygen, air, and laughing gas. The gas source module 10 can be a gas source interface, which is connected with an external gas source or an internal gas source. There can be one or more gas source interfaces. The external gas source provides the gas required by the anesthesia system to the anesthesia system through the gas source interface. In some embodiments, the internal gas source may include an oxygen generator, which provides oxygen to the anesthesia system through a gas source interface (oxygen interface). The internal air source can further include an air pump or air compressor, which provides air to the anesthesia system through a gas source interface (air interface).

Both the anesthesia branch B and the ventilation branch C need to provide the respiratory support for the patient, so that the first gas, which is required for both branches is oxygen-containing gas. The first gas, which is provided by the gas source module 10 to the anesthesia branch B, and the first gas, which is provided by the gas source module 10 to the ventilation branch C can be exactly the same. For example, a mixture of oxygen and air is both provided to the anesthesia branch B and the ventilation branch C. The first gas, which is provided by the gas source module 10 to the anesthesia branch B, and the first gas, which is provided by the gas source module 10 to the ventilation branch C cannot be exactly the same, that is, the same gas source(s) can be connected with the anesthesia branch B and the ventilation branch C, or some same gas source(s) can be connected with the anesthesia branch B and the ventilation branch C. For example, a mixture of oxygen and air is provided to the anesthesia branch B, while oxygen is provided to the ventilation branch C. For further example, air is provided to the anesthesia branch B, while oxygen is provided to the ventilation branch C, wherein both air and oxygen are oxygen-containing gases. In summary, regardless of whether the gas, which enters into the anesthesia branch B and the ventilation branch C from the gas source module 10, is firstly subjected to same or different treatments or to same or different mixing process, it is acceptable as long as the first gas, which is used in the anesthesia branch B and the ventilation branch C contains oxygen. Considering that the anesthesia branch B needs to anesthetize the patient, the gas source module 10 can not only provide the first gas to the anesthesia branch B, but also separately provide laughing gas to the anesthesia branch B.

The oxygen supply branch A includes an oxygen supply apparatus 80, which includes an output port. The oxygen supply apparatus 80 is configured to provide oxygen-containing gas, which is outputted through the output port of the oxygen supply apparatus 80 and humidified to provide first respiratory support for the patient. The oxygen-containing gas, as its name suggests, contains oxygen components, such as a mixture of oxygen and air.

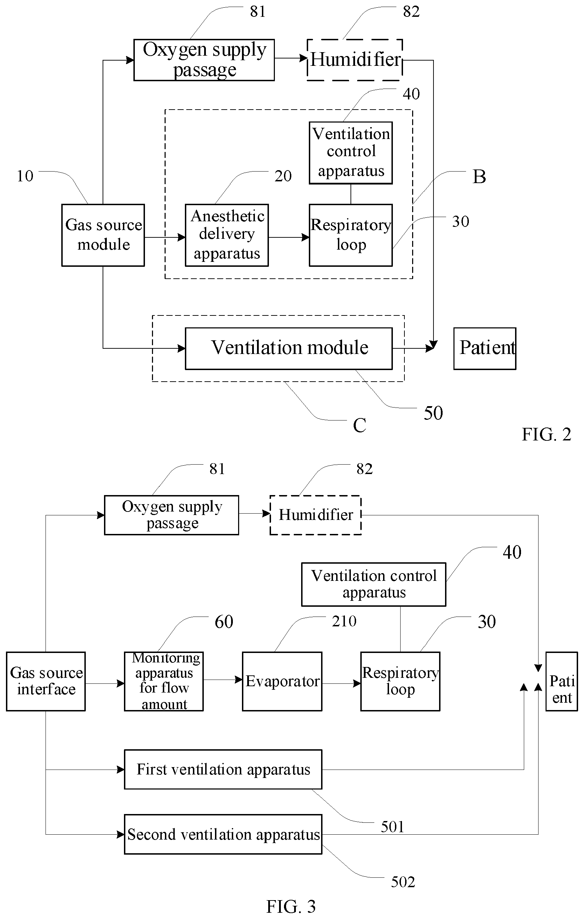

As shown in FIG. 2, the oxygen supply apparatus 80 includes an oxygen supply passage 81 and a control valve for oxygen supply (not shown). The oxygen supply passage 81 is connected with the gas source module 10, so as to obtain the oxygen-containing gas from the gas source module 10. The oxygen-containing gas, which is provided by the gas source module 10, is outputted, through the control valve for oxygen supply, to a humidifier 82, according to a flow amount which is preset by a user. An oxygen concentration of the oxygen-containing gas can also be controlled (such as controlled by the user) to be regulated. The humidifier 82 is configured to humidify the oxygen-containing gas, which is outputted from the oxygen supply passage 81. The oxygen-containing gas provided by the oxygen supply apparatus 80 can assist in oxygen supply to the patient, such as during patient resuscitation and other stages, in which the oxygen supply apparatus 80 can be a conventional auxiliary oxygen supply apparatus in an anesthesia machine. Of course, the oxygen supply apparatus 80 can also provide an oxygen therapy of high-flow mount, that is, the oxygen supply apparatus 80 can deliver oxygen of high flow amount to the patient, thereby achieving preoxygenation of the patient, extending a suffocation time window of the patient, and reserving time for intubation and other operations of the doctor. For example, if a flow amount of the oxygen-containing gas, which is outputted by the oxygen supply apparatus 80, exceeds 15 Liters/minute (such as 20-80 Liters/minute), first respiratory support can be provided to the patient, which can be non-periodic respiratory support. Non-periodic respiratory support refers to a continuous gas delivery to the patient.

The anesthesia branch B is configured to mix the first gas with an anesthetic, so as to obtain a second gas, and configured to provide (such as deliver) the second gas to the patient, so as to provide anesthesia respiratory support for the patient. The anesthesia branch B can include an anesthetic delivery apparatus 20, a respiratory loop 30, and a ventilation control apparatus 40. The gas source module 10 is connected with the respiratory loop 30 through the anesthetic delivery apparatus 20, that is, the three are sequentially connected in a gas path.

The anesthetic delivery apparatus 20 is configured to mix the first gas, which is provided by the gas source module 10, with the anesthetic, so as to obtain a mixed gas (second gas), and configured to control an anesthetic concentration of the mixed gas, and configured to deliver the mixed gas (second gas) to the respiratory loop 30. The anesthetic delivery apparatus 20 includes an evaporator.

The respiratory loop 30 is a gas path which connects the anesthetic delivery apparatus 20 with the patient, and configured to provide (such as deliver) the second gas to the patient. The respiratory loop 30 is capable of recycling the gas exhaled by the patient to save anesthetic and reduce environmental pollution. The respiratory loop 30 can include various connection catheters and output ports, which output ports can be various types of accessories, such as a tracheal intubation tube, a tracheal catheter with a balloon at its end, etc. A gas purification apparatus can be arranged inside the respiratory loop 30, and configured to remove at least a portion of carbon dioxide, which is exhaled by the patient into the respiratory loop. CO₂ absorbent (sodium lime) can be arranged inside the gas purification apparatus. Through a reaction between CO₂ absorbent and CO₂, the goal of removing CO2 is achieved, while generating water and heat through the reaction, which is beneficial for maintaining a temperature and humidity of the gas inhaled by the patient.

The ventilation control apparatus (i.e., anesthesia ventilation control apparatus) 40 is configured to control the respiratory loop 30 to deliver the second gas to the patient through its output port, so as to enable the anesthesia system to provide anesthesia respiratory support for the patient. For example, the ventilation control apparatus 40 controls the respiratory loop to periodically deliver the second gas to the patient, so as to enable the anesthesia system to provide the anesthesia respiratory support which is periodic for the patient. The ventilation control apparatus 40 can automatically or manually (such as balloon) perform an anesthesia ventilation control. For example, the ventilation control apparatus 40 may include multiple valves and a board card for driving the multiple valves. The board card controls the multiple valves to periodically deliver the second gas to the patient, thus providing the anesthesia respiratory support which is periodic.

During an operation of the anesthesia system, a monitoring apparatus for flow amount is configured to implement an operation of gas component mixing on the first gas, which is provided by the gas source module 10. Then an evaporator is configured to implement an anesthetic addition and a concentration adjustment on the first gas, so as to form the second gas. The second gas enters into the respiratory loop 30, and the ventilation control apparatus 40 performs a ventilation control, so as to deliver the second gas to the patient. The exhaust gas exhaled by the patient is purified by the gas purification apparatus and discharged or recycled for use. During the above process, the anesthesia system also monitors a machine state and patient parameter(s), so as to ensure patient safety and issue abnormal alarm(s).

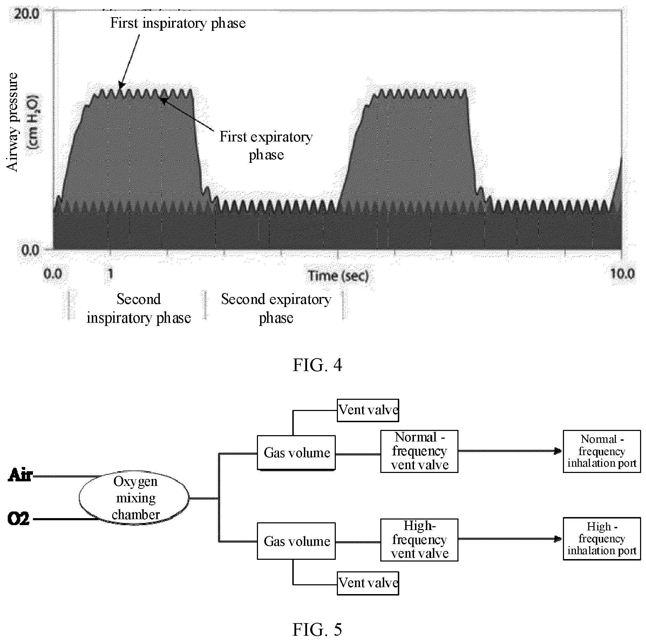

The ventilation branch C includes a ventilation module 50, which is configured to receive the first gas and provide the first gas to the patient at the first ventilation frequency and at the second ventilation frequency, respectively, so as to provide the respiratory support for the patient. The first ventilation frequency and the second ventilation frequency can be the same or different, and this embodiment uses different frequencies for explanation. Waveform(s) of an airway pressure, when the ventilation module 50 provides the respiratory support for patient, is shown in FIG. 4. The two ventilation frequencies correspond to different respiratory cycles, wherein a respiratory cycle which corresponds to the first ventilation frequency includes a first inspiratory phase (first inspiratory stage) and a first expiratory phase (first expiratory state), while a respiratory cycle which corresponds to the second ventilation frequency includes a second inspiratory phase (second inspiratory stage) and a second expiratory phase (second expiratory stage). The respiratory support, which corresponds to two ventilation frequencies, simultaneously acts on the patient for at least a portion of time, that is, when the ventilation module 50 provides the first gas to the patient in multiple second inspiratory phases at the second ventilation frequency, the ventilation module 50 simultaneously provides the first gas to the patient at the first ventilation frequency, in at least one second inspiratory phase of the multiple second inspiratory phases. Thus, the ventilation module 50 provides second respiratory support for the patient at both the first ventilation frequency and the second ventilation frequency simultaneously. It can be seen that in addition to providing existing anesthesia respiratory support, the anesthesia system can also provide superimposed ventilation of two ventilation frequencies through the ventilation module 50, and achieves a selection or a switching of these two functions. That is, the ventilation module 50 can be used to provide the first gas to the patient at both the first ventilation frequency and the second ventilation frequency, when a respiratory support mode which is provided by the anesthesia system, is superimposed ventilation, thereby providing second respiratory support for the patient. This disclosure improves an elimination rate of carbon dioxide, when providing respiratory support for patient, by superposing ventilation modes of two different frequencies.

As shown in FIG. 3, in one embodiment, the ventilation module 50 includes a first ventilation apparatus 501 and a second ventilation apparatus 502.

The first ventilation apparatus 501 is configured to provide the first gas to a patient at a first ventilation frequency, that is, to provide the respiratory support for a patient at a first ventilation frequency by using the first gas. The second ventilation apparatus 502 is configured to provide the first gas to a patient at a second ventilation frequency, that is, to provide the respiratory support for a patient at a second first ventilation frequency by using the first gas. As shown in FIG. 4, the first ventilation frequency is higher than the second ventilation frequency, for example, the first ventilation frequency is not lower than 3Hz, and the second ventilation frequency is lower than 3Hz. The first ventilation frequency and the second ventilation frequency can be pre-set, for example, by the user, and setting ranges of the first ventilation frequency and the second ventilation frequency may overlap. For example, a setting range of the first ventilation frequency is higher than or equal to 50bpm (bpm, unit of beats per minute), and a setting range of the second ventilation frequency is less than or equal to 100 bpm. Considering practical applications, in this embodiment, the setting range of the first ventilation frequency is 50bpm-1500bpm, and the setting range of the second ventilation frequency is 1-100 bpm. When the ventilation module 50 is in operation, the user can set two ventilation frequencies to be the same, or can set one frequency to be higher than the other. Usually, the first ventilation frequency is higher than the second ventilation frequency.

The first ventilation apparatus 501 and the second ventilation apparatus 502 can work independently, or simultaneously under control. "Working simultaneously under control" actually refers to that the ventilation apparatuses are controlled to work cooperatively. For example, the two ventilation apparatuses can work simultaneously throughout an entire process, or work alternatively, as long as in at least one second inspiratory phase, the first ventilation apparatus 501 can simultaneously provide the patient with the first gas at the first ventilation frequency, thereby jointly providing the respiratory support for the patient. A driving pressure, when the first ventilation apparatus 501 provides the respiratory support for the patient, can be lower than a driving pressure, when the second ventilation apparatus 502 provides the respiratory support for the patient. As shown in FIG. 4, the larger waveform has a lower frequency and corresponds to the second ventilation frequency, the smaller waveform has a higher frequency and corresponds to the first ventilation frequency. The first ventilation apparatus 501 provides relatively high frequency ventilation, which generates a lower level of airway pressure, while the second ventilation apparatus 502 provides relatively low frequency (normal-frequency) ventilation, which intermittently increases the airway pressure. The higher a level of increase, the greater a tidal volume. Therefore, the superposition of high-frequency and normal-frequency ventilation effectively improves the elimination rate of carbon dioxide.

The anesthesia system further includes a human-machine interaction apparatus, which is used for human-machine interaction, such as displaying visual information and receiving user input. The human-machine interaction apparatus can include a display, a touch screen, button(s), a keyboard, a mouse, etc. The human-machine interaction apparatus can be specifically configured to receive, based on a user input, a respiratory support mode, which is provided by the anesthesia system.

When the respiratory support mode, which is received by the human-machine interaction apparatus, is high-frequency ventilation, that is, when the respiratory support mode, which is set by the user on the anesthesia system, is high-frequency ventilation, the first ventilation apparatus 501 is started to provide the first gas to the patient at the first ventilation frequency, so as to provide the respiratory support for the patient. It can be seen that, the first ventilation apparatus 501 can be started individually, so as to provide the patient with relatively high-frequency ventilation.

When the respiratory support mode, which is received by the human-machine interaction apparatus, is normal-frequency ventilation, that is, when the respiratory support mode, which is set by the user on the anesthesia system, is normal-frequency ventilation, the second ventilation apparatus 502 is started to provide the first gas to the patient at the second ventilation frequency, so as to provide the respiratory support for the patient. The first ventilation frequency is higher than the second ventilation frequency. It can be seen that the second ventilation apparatus 502 can be started individually, so as to provide the patient with relatively low-frequency ventilation.

When the respiratory support mode, which is received by the human-machine interaction apparatus, is superimposed ventilation, that is, when the respiratory support mode, which is set by the user on the anesthesia machine, is superimposed ventilation, the first ventilation apparatus 501 and the second ventilation apparatus 502 work simultaneously. The first ventilation apparatus 501 and the second ventilation apparatus 502 provide the first gas to the patient at both first ventilation frequency and second ventilation frequency, thereby providing the respiratory support for the patient.

When the respiratory support mode, which is received by the human-machine interaction apparatus, is anesthesia respiratory support, the anesthesia branch is started to provide the anesthesia respiratory support for the patient. When the respiratory support mode, which is received by the human-machine interaction apparatus, is first respiratory support, the oxygen supply branch is started to provide auxiliary oxygen support for the patient.

It can be seen that, selecting (inputting), by the user, a respiratory support mode, is equivalent to selecting a working mode of the anesthesia system, which makes uses of the different functions of the anesthesia system conveniently.

When the first ventilation apparatus 501 and the second ventilation apparatus 502 provide the respiratory support for the patient, for example, the first ventilation apparatus 501 and the second ventilation apparatus 502 may respectively provide different flow amounts of the first gas to the patient in two different time periods of a respiratory cycle, wherein a flow amount in one time period is lower than a flow amount in the other time period, or a flow amount in one time period is zero. Providing different flow amounts of the first gas in two different time periods is equivalent to switching flow amounts, and a frequency of such switching is the ventilation frequency mentioned above. The two different time periods of a respiratory cycle can be an inspiratory period (inspiratory phase) and an expiratory period (expiratory phase). That is, providing different flow amounts of the first gas to the patient in two different time periods of a respiratory cycle can be providing different flow amounts of the first gas to the patient in the inspiratory period and the expiratory period of a respiratory cycle, wherein the flow amount provided in the inspiratory period is greater than the flow amount provided in the expiratory period, and the flow amount provided in the expiratory period can be zero or not. Of course, in some embodiments, the two different time periods of a respiratory cycle can also be other time periods, that is, two time periods that are not divided by inhalation and exhalation.

The oxygen supply apparatus 80 and the ventilation module 50 can be controlled to work simultaneously, which means that the oxygen supply apparatus 80 can work simultaneously with one or both of the first ventilation apparatus 501 and the second ventilation apparatus 502. When the oxygen supply apparatus 80 and the ventilation module 50 work simultaneously, the oxygen-containing gas, which is outputted by the oxygen supply apparatus 80, is humidified and used to humidify the first gas, which is outputted by the ventilation module 50. Specifically, the oxygen supply apparatus 80 can work simultaneously with the ventilation module 50. When the oxygen supply apparatus 80 and the ventilation module 50 work simultaneously, the oxygen-containing gas, which is outputted from the output port of the oxygen supply apparatus 80, is humidified by the humidifier, so as to form a humidified mixed gas with the first gas, which is outputted from the output port of the ventilation module 50, wherein the humidified mixed gas is outputted to the patient. This disclosure creatively integrates a ventilation module that can provide respiratory support into an anesthesia system, such as on an anesthesia system. In this way, the anesthesia system can provide respiratory support through the ventilation module, and can use the oxygen-containing gas, which is provided by the oxygen supply apparatus and humidified, to humidify an output of the ventilation module, and can further provide anesthesia respiratory support through the ventilation control apparatus 40. For example, in some specific scenarios, such as when an inhalation anesthesia function of the anesthesia system is not required, the respiratory support can be provided to the patient through the ventilation module 50 of the anesthesia system, which improves the application range of the anesthesia system and eliminates needs for additional devices, and eliminates management problems of multiple devices.

The gas interface included by the gas source module 10 is connected with an external gas source. The gas source interface includes an oxygen interface and/or an air interface, and correspondingly, the first gas includes oxygen and/or air. The first ventilation apparatus 501 is connected with the gas source interface, and the first gas is delivered from the gas source interface to the first ventilation apparatus 501 through a first flow path. The second ventilation apparatus 502 is connected with the gas source interface, and the first gas is delivered from the gas source interface to the second ventilation apparatus 502 through a second flow path, wherein the first flow path can be independent of the second flow path. In this way, when the first ventilation apparatus 501 and the second ventilation apparatus 502 work simultaneously, regardless of their flow amount(s) and pressure(s), they do not affect each other, and no decrease exists in the flow amount(s) and pressure(s).

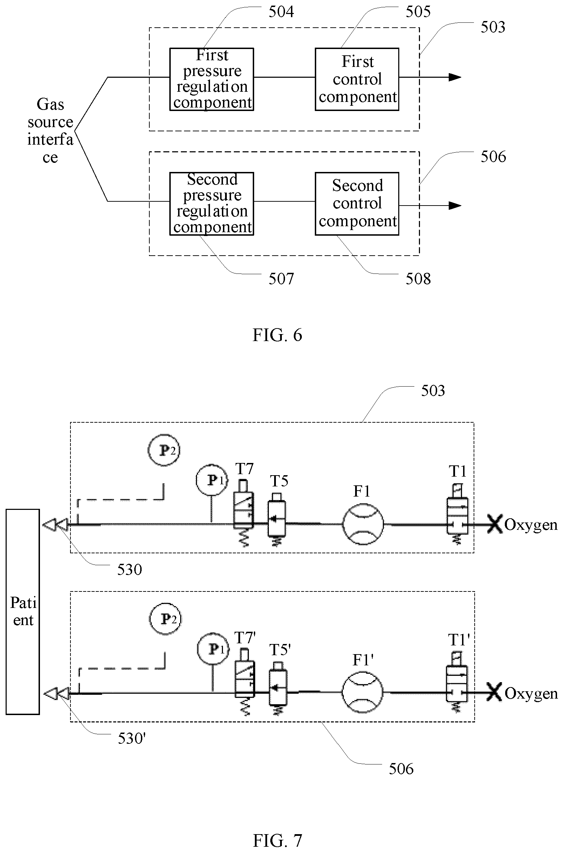

As shown in FIG. 6, the first ventilation apparatus 501 includes a first gas delivery branch 503, which is arranged with a first pressure regulation component 504 for controlling a gas pressure, which is provided by the first ventilation apparatus 501. That is, the first pressure regulation component 504 can regulate the driving pressure, and can be a pressure regulation valve, etc. The pressure regulation valve controls a gas pressure, which is provided by the first ventilation apparatus 501, by regulating an opening degree. Similarly, the second ventilation apparatus 502 includes a second gas delivery branch 506, which is arranged with a second pressure regulation component 507 for controlling a gas pressure, which is provided by the second ventilation apparatus. That is, the second pressure regulation component 507 can regulate the driving pressure, and can be a pressure regulation valve, etc. The pressure regulation valve controls the gas pressure, which is provided by the second ventilation apparatus 502 by regulating an opening degree. It can be seen that, the two ventilation apparatuses have their own pressure regulation components, and there is no need to use a gas volume for pressure regulation. The pressure regulations in the two ventilation apparatuses do not affect each other, and the driving pressure is regulated by a pressure regulation valve, which is fast and sensitive.

The first gas delivery branch 503 is also arranged with a first control component 505, which is located downstream of the first pressure regulation component 504. "Downstream" refers to a direction in which a gas flows, and means that the first gas first passes through the first pressure regulation component 504 and then through the first control component 505. The first control component 504 is configured to control an oxygen concentration of the first gas which is outputted by the first ventilation apparatus 501, and can be a proportional valve or the likes. The second gas delivery branch 506 is also arranged with a second control component 508, which is located downstream of the second pressure regulation component 507. That is, the first gas first passes through the second pressure regulation component 507 and then through the second control component 508. The second control component 508 is configured to control an oxygen concentration of the first gas which is outputted by the second ventilation apparatus 502, and can be a proportional valve or the likes. It can be seen that, the two ventilation apparatuses have their own control components, and there is no need to use an oxygen mixing chamber to regulate the oxygen concentration, and no low flow amount and pressure are resulted.

Compositions of the first gas, which corresponds to different gas delivery branches, are different as explained below.

In one embodiment, the first gas may include oxygen. The gas interface included by the gas source module 10 is an oxygen interface. The oxygen interface is connected with an external oxygen source, such as oxygen delivery pipelines and cylinders in hospitals, or an internal oxygen source, such as a build-in oxygen generator in the anesthesia system. As shown in FIG. 7, the first gas delivery branch 503 is a first oxygen branch, and the first ventilation apparatus 501 further includes a first output port 530. An input end of the first oxygen branch is connected with the oxygen interface, for example, the input end of the first oxygen branch is connected with the oxygen interface through an independent pipeline with no gas volume for pressure regulation on the pipeline. An output end of the first oxygen branch is connected with the first output port 530. The first pressure regulation component 504 is a first pressure regulation valve T1, which is configured to control an oxygen pressure inside the first oxygen branch.

The second gas delivery branch 506 is a second oxygen branch, and the second ventilation apparatus 502 further includes a second output port 530'. An input end of the second oxygen branch is connected with the oxygen interface, for example, the input end of the second oxygen branch is connected with the oxygen interface through an independent pipeline with no gas volume for pressure regulation on the pipeline. Therefore, the oxygen, which is inputted from the oxygen interface is delivered to the first oxygen branch and the second oxygen branch through different pipelines, and the two oxygen branches do not affect each other. An output end of the second oxygen branch is connected with the second output port 530'. The second pressure regulation component 507 is a third pressure regulation valve T1', which is configured to control an oxygen pressure inside the second oxygen branch. The first pressure regulation valve T1 and the third pressure regulation valve T1' can be mechanical or electronic pressure regulation valves. The latter can be explained as an example in this embodiment, and the electronic pressure regulation valve can automatically regulate according to setting(s).

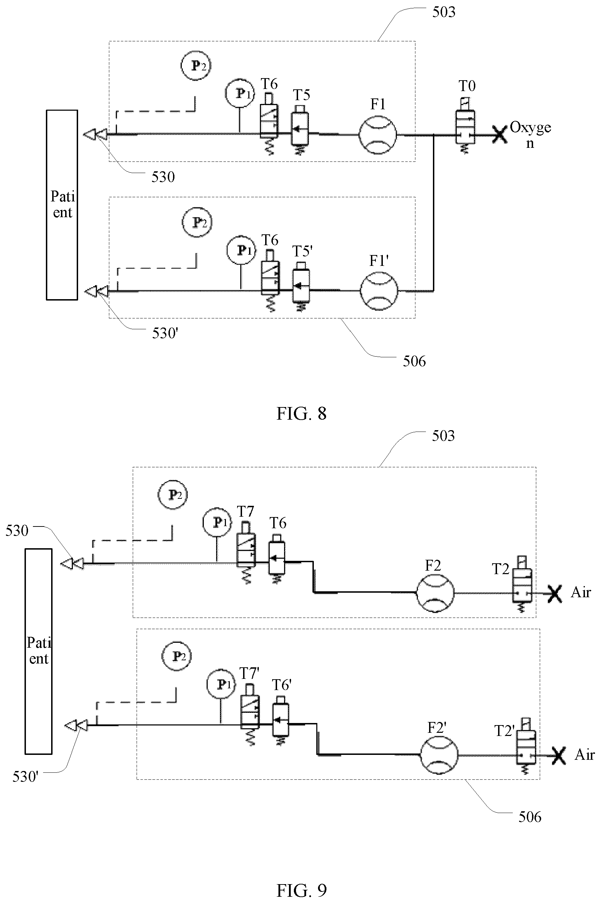

In some embodiments, two oxygen branches do not need to be arranged with one pressure regulation valve each, but instead share one pressure regulation valve. For example, as shown in FIG. 8, the anesthesia system includes a fifth pressure regulation valve T0, which is configured to control a gas pressure. The first ventilation apparatus 501 includes a first oxygen branch and a first output port 530. An input end of the first oxygen branch is connected with the oxygen interface through the fifth pressure regulation valve T0. An output end of the first oxygen branch is connected with the first output port 530. The second ventilation apparatus 502 includes a second oxygen branch and a second output port 530'. An input end of the second oxygen branch is connected with the oxygen interface through the fifth pressure regulation valve T0. An output end of the second oxygen branch is connected with the second output port 530'. There is no gas volume for pressure regulation between the fifth pressure regulation valve T0 and the oxygen interface. Although the two oxygen branches share the same pressure regulation valve, due to direct connection to the oxygen interface with a constant pressure and flow amount, there is no situation of low flow amount and pressure, when the two oxygen branches are used. Moreover, using a pressure regulation valve to regulate the driving pressure is fast and sensitive.

The first control component 504 inside the first oxygen branch is a first control valve T5, which is located downstream of the first pressure regulation valve T1 or the fifth pressure regulation valve T0. That is oxygen first passes through the first pressure regulation valve T1 or the fifth pressure regulation valve T0 and then passes through the first control valve T5. The first control valve T5 is configured to control a flow amount of oxygen inside the first oxygen branch and enable the oxygen inside the first oxygen branch to form a gas flow of a first ventilation frequency, that is, to enable the oxygen inside the first oxygen branch to be provided to the patient at the first ventilation frequency. The first control valve T5 is controlled to switch between large flow amount(s) and small flow amount(s) at a preset first ventilation frequency, so as to enable the first gas, which is outputted from the first output port 530, to form a high-frequency gas flow. The term "high-frequency gas flow" used in this disclosure refers to a gas with an output frequency, which is greater than a certain critical value, such as a gas with an output frequency, which is greater than or equal to 2Hz, or a gas with an output frequency, which is greater than or equal to 3Hz. The ventilation frequency (switching frequency of flow amount(s) or switching frequency of opening size(s) of valve) of the first control valve T5 can be pre-set. The first control valve T5 is specifically used for high-frequency ventilation control (such as switching between two different valve flow amounts of one greater flow amount and one smaller flow amount, at a high frequency, which frequency is 2Hz or above, or 3Hz or above, such as between 3-20Hz), so as to enable the first gas to form a high-frequency pulse gas flow, which flow provides high-frequency jet ventilation to the patient after passing through a jet accessory, which is connected with the first output port 530. If the first output port 530 is not connected with the jet accessory, the high-frequency pulse gas flow can provide high-frequency ventilation to the patient. The first control valve T5 can be an electromagnetic valve, an electromagnetic servo valve, an electromagnetic switch valve, or an electromagnetic proportional valve, etc.

The second control component 508 inside the second oxygen branch is a third control valve T5', which is located downstream of the third pressure regulation valve T1' or the fifth pressure regulation valve T0. That is oxygen first passes through the third pressure regulation valve T 1' or the fifth pressure regulation valve T0 and then passes through the third control valve T5'. The third control valve T5'is configured to control a flow amount of oxygen inside the second oxygen branch and enable the oxygen inside the second oxygen branch form a gas flow of a second ventilation frequency, that is, to enable the oxygen inside the second oxygen branch to be provided to the patient at the second ventilation frequency. Similarly, the third control valve T5' is controlled to switch between large flow amount(s) and small flow amount(s) at a preset second ventilation frequency, so as to enable the first gas, which is outputted from the second output port 530, to form a normal-frequency (or low-frequency) gas flow. The term "normal-frequency gas flow" used in this disclosure refers to a gas with an output frequency, which is lower than a certain critical value, such as a gas with an output frequency, which is lower than 3Hz. The ventilation frequency (switching frequency of flow amount(s) or switching frequency of opening size(s) of valve) of the third control valve T5' can be pre-set. The third control valve T5' is specifically used for normal-frequency ventilation control (such as switching between two different valve flow amounts of one greater flow amount and one smaller flow amount according to the second ventilation frequency, which frequency is lower than 3Hz, such as between 10-50bpm), so as to enable the first gas to form a normal-frequency pulse gas flow, which flow provides normal-frequency jet ventilation to the patient after passing through a jet accessory, which is connected with the second output port 530'. If the second output port 530' is not connected with the jet accessory, the normal-frequency pulse gas flow can provide norma-frequency ventilation to the patient. The third control valve T5' can be an electromagnetic valve, an electromagnetic servo valve, an electromagnetic switch valve, or an electromagnetic proportional valve, etc.

In one embodiment, the first gas may include air. The gas interface included by the gas source module 10 is an air interface. The air interface is connected with an external air source, such as air delivery pipelines and cylinders in hospitals, or an internal air source, such as air pumps and air compressors. As shown in FIG. 9, the first gas delivery branch 503 is a first air branch, and the first ventilation apparatus 501 further includes a first output port 530. An input end of the first air branch is connected with the air interface, for example, the input end of the first air branch is connected with the air interface through an independent pipeline with no gas volume for pressure regulation on the pipeline. An output end of the first air branch is connected with the first output port 530. The first pressure regulation component 504 is a second pressure regulation valve T2, which is configured to control an air pressure inside the first air branch.

The second gas delivery branch 506 is a second air branch, and the second ventilation apparatus 502 further includes a second output port 530'. An input end of the second air branch is connected with the air interface, for example, the input end of the second air branch is connected with the air interface through an independent pipeline with no gas volume for pressure regulation on the pipeline. Therefore, the air, which is inputted from the air interface is delivered to the first air branch and the second air branch through different pipelines, and the two air branches do not affect each other. An output end of the second air branch is connected with the second output port 530'. The second pressure regulation component 507 is a fourth pressure regulation valve T2', which is configured to control an air pressure inside the second air branch. Similarly, the second pressure regulation valve T2 and the fourth pressure regulation valve T2' can be mechanical or electronic pressure regulation valves.

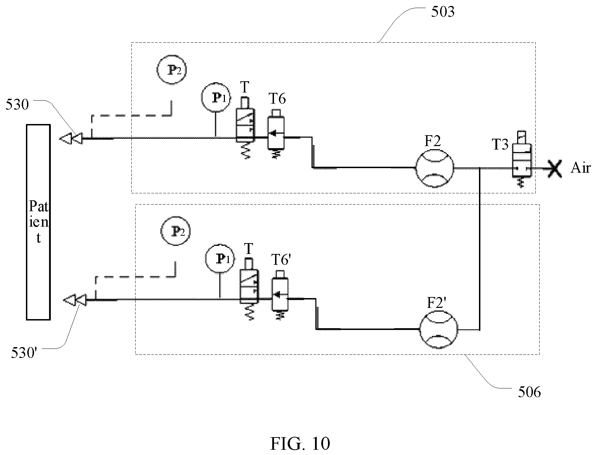

In some embodiments, two air branches do not need to be arranged with one pressure regulation valve each, but instead share one pressure regulation valve. For example, as shown in FIG. 10, the anesthesia system includes a sixth pressure regulation valve T3, which is configured to control a gas pressure. The first ventilation apparatus 501 includes a first air branch and a first output port 530. An input end of the first air branch is connected with the air interface through the sixth pressure regulation valve T3. An output end of the first air branch is connected with the first output port 530. The second ventilation apparatus 502 includes a second air branch and a second output port 530'. An input end of the second air branch is connected with the air interface through the sixth pressure regulation valve T3. An output end of the second air branch is connected with the second output port 530'. There is no gas volume for pressure regulation between the sixth pressure regulation valve T3 and the air interface. Although the two air branches share the same pressure regulation valve, due to direct connection to the air interface with a constant pressure and flow amount, there is no situation of low flow amount and pressure, when the two air branches are used. Moreover, using a pressure regulation valve to regulate the driving pressure is fast and sensitive.

The first control component 504 inside the first air branch is a second control valve T6, which is located downstream of the second pressure regulation valve T2 or the sixth pressure regulation valve T3. That is, air first passes through the e second pressure regulation valve T2 or the sixth pressure regulation valve T3 and then passes through the second control valve T6. The second control valve T6 is configured to control a flow amount of air inside the first air branch and enable the air inside the first air branch to form a gas flow of a first ventilation frequency, that is, to enable the air inside the first air branch to be provided to the patient at the first ventilation frequency. The functions of the second control valve T6 are the same as that of the first control valve T5 mentioned above, and are not elaborated here. The second control valve T6 can be an electromagnetic valve, an electromagnetic servo valve, an electromagnetic switch valve, or an electromagnetic proportional valve, etc.

The second control component 508 inside the second air branch is a fourth control valve T6', which is located downstream of the fourth pressure regulation valve T2' or the sixth pressure regulation valve T3. That is, air first passes through the fourth pressure regulation valve T2' or the sixth pressure regulation valve T3 and then passes through the fourth control valve T6'. The fourth control valve T6' is configured to control a flow amount of air inside the second air branch and enable the air inside the second air branch form a gas flow of a second ventilation frequency, that is, to enable the air inside the second air branch to be provided to the patient at the second ventilation frequency. The functions of the fourth control valve T6' are the same as that of the third control valve T5' mentioned above, and are not elaborated here. The fourth control valve T6' can be an electromagnetic valve, an electromagnetic servo valve, an electromagnetic switch valve, or an electromagnetic proportional valve, etc.

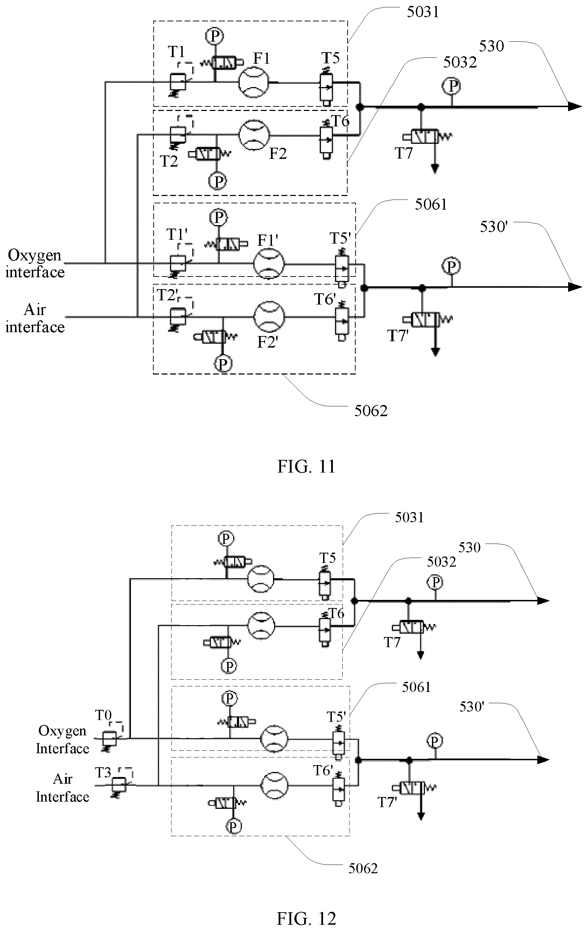

In one embodiment, the first gas may include air and oxygen, i.e., the first gas is a mixture of air and oxygen. The gas interface included by the gas source module 10 is an oxygen interface and an air interface. As shown in FIG. 11, the first gas delivery branch 503 includes a first oxygen branch 5031 and a first air branch 5032. An input end of the first oxygen branch 5031 is connected with the oxygen interface, for example, the input end of the first oxygen branch is connected with the oxygen interface through an independent pipeline with no gas volume for pressure regulation on the pipeline. An input end of the first air branch 5032 is connected with the air interface, for example, the input end of the first air branch 5032 is connected with the air interface through an independent pipeline with no gas volume for pressure regulation on the pipeline. The first oxygen branch 5031 and the first air branch 5032 are converged with each other, and then connected with the first output port 530. The first pressure regulation component 504 includes a first pressure regulation valve T1 and a second pressure regulation valve T2. The first oxygen branch is arranged with the first pressure regulation valve T1, which is configured to control an oxygen pressure inside the first oxygen branch. The first air branch is arranged with the second pressure regulation valve T2, which is configured to control a pressure of the air inside the first air branch. The first pressure regulation valve T1 and the second pressure regulation valve T2 can work together to determine the driving pressure of the first ventilation apparatus for the respiratory support. The first oxygen branch 5031 and the first air branch 5032 are respectively connected with the gas source interface through pipelines, and are arranged with independent control components. Accordingly, there is no need to use an oxygen mixing chamber to adjust the oxygen concentration, and no low flow amount and pressure are resulted.

The second gas delivery branch 506 includes a second oxygen branch 5061 and a second air branch 5062. An input end of the second oxygen branch 5061 is connected with the oxygen interface. An input end of the second air branch 5062 is connected with the air interface. The second oxygen branch 5061 and the second air branch 5062 are converged with each other, and then connected with the second output port 530'. The second oxygen branch 5061 is arranged with a third pressure regulation valve T1', which is configured to control an oxygen pressure inside the second oxygen branch. The second air branch is arranged with a fourth pressure regulation valve T2', which is configured to control an air pressure inside the second air branch. The third pressure regulation valve T1' and the fourth pressure regulation valve T2' can work together to determine the driving pressure of the first ventilation apparatus for the respiratory support. The second oxygen branch 5061 and the second air branch 5062 are respectively connected with the gas source interface through pipelines with no gas volume for pressure regulation on the pipeline, and are arranged with independent control components. Accordingly, there is no need to use an oxygen mixing chamber to adjust the oxygen concentration, and no low flow amount and pressure are resulted.

In some embodiments, two oxygen branches do not need to be arranged with one pressure regulation valve each, but instead share one pressure regulation valve; two air branches do not need to be arranged with one pressure regulation valve each, but instead share one pressure regulation valve. For example, as shown in FIG. 12, the anesthesia system includes a fifth pressure regulation valve T0 and a sixth pressure regulation valve T3, both of which are configured to control a gas pressure. The first ventilation apparatus includes a first oxygen branch 5031, a first air branch 5032, and a first output port 530. An input end of the first oxygen branch 5031 is connected with the oxygen interface through the fifth pressure regulation valve T0, and an input end of the first air branch 5032 is connected with the air interface through the sixth pressure regulation valve T3. The first oxygen branch 5031 and the first air branch 5032 are converged with each other and then connected with the first output port 530.

The second ventilation apparatus includes a second oxygen branch 5061, a second air branch 5062, and a second output port 530'. An input end of the second oxygen branch 5061 is connected with the oxygen interface through the fifth pressure regulation valve T0, and an input end of the second air branch 5062 is connected with the air interface through the sixth pressure regulation valve T3. The second oxygen branch 5061 and the second air branch 5062 are converged with each other and then connected with the second output port 530'.

The first oxygen branch 5031 is arranged with a first control valve T5, which is located downstream of the first pressure regulation valve T 1 or the fifth pressure regulation valve T0 (referring FIGS. 11 and 12 respectively). That is, oxygen first passes through the first pressure regulation valve T1 or the fifth pressure regulation valve T0, and then passes through the first control valve T5. The first control valve T5 is configured to control a flow amount of oxygen inside the first oxygen branch 5031 and to enable the oxygen inside the first oxygen branch 5031 to form a gas flow of a first ventilation frequency, that is, to enable the oxygen inside the first oxygen branch 5031 to be provided to the patient at the first ventilation frequency. The first air branch 5032 is arranged with a second control valve T6, which is located downstream of the second pressure regulation valve T2 or the sixth pressure regulation valve T3. That is, air first passes through the second pressure regulation valve T2 or the sixth pressure regulation valve T3 and then passes through the second control valve T6. The second control valve T6 is configured to control a flow amount of air inside the first air branch 5032 and to enable the air inside the first air branch 5032 to form a gas flow of a first ventilation frequency, that is, to enable the air inside the first air branch 5032 to be provided to the patient at the first ventilation frequency. By controlling an opening degree of the first control valve T5 and the second control valve T6, an oxygen concentration of the first ventilation apparatus 501 is set. The first control valve T5 and the second control valve T6 are controlled to switch between large flow amount(s) and small flow amount(s) at a preset first ventilation frequency, so as to enable the first gas, which is outputted from the first output port 530, to form a high-frequency gas flow. The specific functions have been elaborated in detail in the aforementioned embodiments and are not elaborated here.

The second oxygen branch 5061 is arranged with a third control valve T5', which is located downstream of the third pressure regulation valve T 1' or the fifth pressure regulation valve T0. That is, oxygen first passes through the third pressure regulation valve T1' or the fifth pressure regulation valve T0 and then passes through the third control valve T5'. The third control valve T5' is configured to control a flow amount of oxygen inside the second oxygen branch 5061 and to enable the oxygen inside the second oxygen branch 5061 to form a gas flow of a second ventilation frequency, that is, to enable the oxygen inside the second oxygen branch 5061 to be provided to the patient at the second ventilation frequency. The second air branch 5062 is arranged with a fourth control valve T6', which is located downstream of the fourth pressure regulation valve T2' or the sixth pressure regulation valve T3. That is, air first passes through the fourth pressure regulation valve T2' or the sixth pressure regulation valve T3, and then passes through the fourth control valve T6'. The fourth control valve T6' is configured to control a flow amount of air inside the second air branch and enable the air inside the second air branch to form a gas flow of a second ventilation frequency, that is, to enable the air inside the second air branch to be provided to the patient at the second ventilation frequency. By controlling an opening degree of the third control valve T5' and the fourth control valve T6', an oxygen concentration of the second ventilation apparatus 502 is set. The third control valve T5' and the fourth control valve T6' are controlled to switch between large flow amount(s) and small flow amount(s) at a preset second ventilation frequency, so as to enable the first gas, which is outputted from the second output port 530, to form a normal-frequency gas flow. The specific functions have been elaborated in detail in the aforementioned embodiments and are not elaborated here.

In the embodiments of FIGS. 11 and 12, the two control valves of each ventilation apparatus are used for both flow amount control and switching between large flow amount(s) and small flow amount(s) at a preset frequency. In other embodiments, the above two control valves of each ventilation apparatus can be used only for flow amount control, while a control valve for switching between large flow amount(s) and small flow amount(s) at a preset frequency is set downstream of a converged node of the oxygen and air branches. Specifically, the first control valve T5 and the second control valve T6 of the first ventilation apparatus are used for the flow amount control of oxygen and the flow amount control of air, respectively; and a fifth control valve is arranged downstream of the converged node of the first oxygen branch and the first air branch. In this embodiment, the first control component includes a first control valve T5, a second control valve T6, and a fifth control valve. The fifth control valve is configured to enable the first gas to form a gas flow of a first ventilation frequency, that is, to control a flow amount of the first gas, which is formed by mixing the air and oxygen, and to enable said first gas to be provided to the patient at the first ventilation frequency. The fifth control valve is controlled to switch between large flow amount(s) and small flow amount(s) at a preset first ventilation frequency, so as to enable the first gas, which is outputted from the first output port 530, to form a high-frequency gas flow. The ventilation frequency (switching frequency of flow amount(s) or switching frequency of opening size(s) of valve) of the fifth control valve can be pre-set. The fifth control valve is specifically used for high-frequency ventilation control (such as switching between two different valve flow amounts of one greater flow amount and one smaller flow amount, at a high frequency, which frequency is 2Hz or above, or 3Hz or above, such as between 3-20Hz), so as to enable the first gas to form a high-frequency pulse gas flow, which flow provides high-frequency jet ventilation to the patient after passing through a jet accessory, which is connected with the first output port 530. If the first output port 530 is not connected with the jet accessory, the high-frequency pulse gas flow can provide high-frequency ventilation to the patient. The fifth control valve can be an electromagnetic valve, an electromagnetic servo valve, an electromagnetic switch valve, or an electromagnetic proportional valve, etc.

Similarly, the third control valve T5' and the fourth control valve T6' of the second ventilation apparatus are used for the flow amount control of oxygen and the flow amount control of air, respectively, and a sixth control valve is arranged downstream of the converged node of the second oxygen branch and the second air branch. In this embodiment, the second control component includes a third control valve T5', a fourth control valve T6', and a sixth control valve. The sixth control valve is configured to enable the first gas to form a gas flow of a second ventilation frequency, that is, to control a flow amount of the first gas, which is formed by mixing the air and oxygen, and to enable said first gas to be provided to the patient at the second ventilation frequency. The sixth control valve is controlled to switch between large flow amount(s) and small flow amount(s) at a preset second ventilation frequency, so as to enable the first gas, which is outputted from the second output port 530, to form a normal-frequency (or low-frequency) gas flow. The ventilation frequency (switching frequency of flow amount(s) or switching frequency of opening size(s) of valve) of the sixth control valve can be pre-set. The sixth control valve is specifically used for normal-frequency ventilation control (such as switching between two different valve flow amounts of one greater flow amount and one smaller flow amount at a second ventilation frequency, which frequency is lower than 3Hz, such as between 10-50bpm), so as to enable the first gas to form a normal-frequency pulse gas flow, which flow provides normal-frequency jet ventilation to the patient after passing through a jet accessory, which is connected with the second output port 530'. If the second output port 530' is not connected with the jet accessory, the normal-frequency pulse gas flow can provide normal-frequency ventilation to the patient. The sixth control valve can be an electromagnetic valve, an electromagnetic servo valve, an electromagnetic switch valve, or an electromagnetic proportional valve, etc.

As shown in FIGS. 7, 8, 11, and 12, a first safety valve T7 for pressure relief can also be provided between the first control valve T5 and the first output port 530. At least one pressure sensor for monitoring pressure is also arranged between the first control valve T5 and the first output port 530. The first safety valve T7 can be specifically configured to connect the first output port 530 with an exhaust port of the first safety valve T7, when a pressure monitored by the pressure sensor exceeds a preset threshold, so that the gas between the first output port 530 and the first safety valve T7 is discharged through the exhaust port, which prevents the patient from developing barotrauma. The first safety valve T7 can also be configured to separate airways between the output port 530 and the converged node, when a pressure monitored by the pressure sensor exceeds a preset threshold. For example, the first safety valve T7 is a three-way valve, a first port of the three-way valve is connected with the first output port 530, a second port of the three-way valve is connected with the converged node, and a third port of the three-way valve is the exhaust port. Under normal circumstances, the first port of the three-way valve is connected (communicated) with the second port of the three-way valve, while the first port of the three-way valve is disconnected from the exhaust port, and the second port of the three-way valve is disconnected from the exhaust port. When the pressure monitored by the pressure sensor exceeds a preset threshold, the three-way valve disconnects the first port from the second port and connects(communicates) the first port with the exhaust port, thereby releasing the gas with excessive pressure and improving safety. The preset threshold can be set based on clinical experience or doctor requirements.

Similarly, a second safety valve T7' for pressure relief is also provided between the third control valve T5' and the second output port 530'. At least one pressure sensor for monitoring pressure is also arranged between the third control valve T5' and the second output port 530'. The second safety valve T7' can be specifically configured to connect the second output port 530' to an exhaust port of the second safety valve T7', when a pressure monitored by the pressure sensor exceeds a preset threshold, so that the gas between the second output port 530' and the second safety valve T7' is discharged through the exhaust port, which prevents the patient from developing barotrauma. It can be seen that the first and second ventilation apparatuses can adopt the same gas path structure.

In the embodiments of FIGS. 9, 10, 11, and 12, a third safety valve for pressure relief can also be provided between the second control valve T6 and the first output port 530. At least one pressure sensor for monitoring pressure is also arranged between the second control valve T6 and the first output port 530. The third safety valve can be specifically configured to connect the first output port 530 with an exhaust port of the third safety valve, when a pressure monitored by the pressure sensor exceeds a preset threshold, so that the gas between the first output port 530 and the third safety valve is discharged through the exhaust port, which prevents the patient from developing barotrauma. A fourth safety valve for pressure relief can also be arranged between the fourth control valve T6' and the second output port 530'. At least one pressure sensor for monitoring pressure is also arranged between the fourth control valve T6'and the second output port 530'. The fourth safety valve can be specifically configured to connect the second output port 530' with an exhaust port of the fourth safety valve, when a pressure monitored by the pressure sensor exceeds a preset threshold, so that the gas between the second output port 530' and the fourth safety valve is discharged through the exhaust port, which prevents the patient from developing barotrauma.

In the above embodiment, a first flow amount sensor F1 (F 1') for monitoring a flow amount of the oxygen branch can also be arranged inside the two oxygen branches. A second flow amount sensor F2 (F2') for monitoring a flow amount of the air branch can also be arranged inside the two air branches 520. Through these flow amount sensors, the flow amounts of oxygen and air can be well monitored.

From the above first ventilation apparatus, it can be seen that the high-frequency gas flow formed at the first output port 530 of the first ventilation apparatus can ventilate the patient at a frequency of at least 3Hz, or in some embodiments, the high-frequency gas flow formed at the first output port 530 of the first ventilation apparatus can ventilate the patient at any one frequency of 50-1500bpm. High frequency ventilation includes high-frequency positive pressure ventilation, high-frequency oscillation ventilation, and high-frequency jet ventilation. When the first ventilation apparatus mentioned above is used for high-frequency positive pressure ventilation or high-frequency oscillation ventilation, the ventilation frequency for the patient can be at least 3Hz. When the first ventilation apparatus is used for high-frequency jet ventilation, the ventilation frequency for the patient can be 50-1500bpm. The high-frequency ventilation provided by the first ventilation apparatus can be pure high-frequency ventilation, or high-frequency jet ventilation, which is formed by adding a jet accessory. Specifically, if the first output port 530 is not connected with the jet accessory but with other ventilation accessories (such as trachea, intubation, mask, nose mask, etc.), the first ventilation apparatus only provides high-frequency ventilation for the patient. If the first output port 530 is connected with a jet accessory, the first ventilation apparatus can further provide high-frequency jet ventilation for the patient. Take the later for example, the first output port 530 can be connected with the jet accessory, and the high-frequency gas flow, which is outputted by the first output port 530 is delivered through the injection accessory to form a gas flow of high-frequency jet. The jet accessory can be provided by a third-party manufacturer, and the user can connect it to the first output port 530. Of course, the jet accessory can also a part of anesthesia system accessories, that is, the anesthesia system includes a jet accessory which is connected with the first ventilation apparatus. The jet accessory receives the first gas outputted by the first ventilation apparatus and outputs the first gas as a gas flow of high-frequency jet. That is, the first output port 530 outputs a high-frequency gas flow (gas flow of high-frequency pulse), which becomes a gas flow of high-frequency jet after passing through the jet accessory. The ventilation frequency of the gas flow of high-frequency jet for ventilating the patient can be 3 Hz or above, or between 50-1500bpm, thus providing the patient with high-frequency jet ventilation. The jet accessory includes a jet channel, an output aperture of the jet channel is less than 4mm. The jet accessory may include a jet nozzle, a jet needle, or a stent (such as a tracheoscope, a bronchoscope, a scope sheath, etc.). The stent is configured to support human tissue or connect the endotracheal tube to facilitate the surgery of the doctor. A wall of the stent is arranged with a hole for the gas jet. (For example, its aperture is less than 4mm).

Similarly, the second output port 530' of the second ventilation apparatus can be connected with a jet accessory to provide normal-frequency jet ventilation to the patient, or can provide to the patient normal-frequency positive pressure ventilation or normal-frequency oscillation ventilation without connecting the jet accessory, which is similar to the function of the first ventilation apparatus, but with a lower frequency, and not elaborated here.

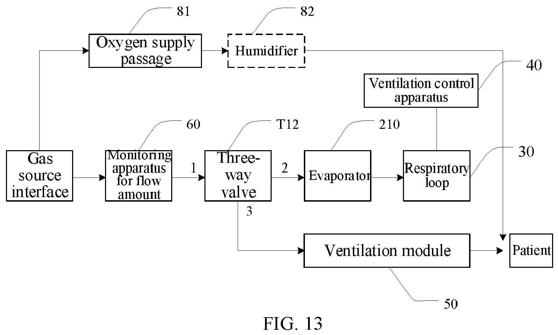

As shown in FIGS. 3 and 13, in some embodiments, the anesthesia branch further includes a monitoring apparatus for flow amount 60, which is configured to control (regulate) a flow amount of the first gas. The monitoring apparatus for flow amount 60 can be mechanical or fully electronic. The monitoring apparatus for flow amount 60 can also be configured to detect the flow amount of the first gas. There are multiple connection methods between the monitoring apparatus for flow amount 60, the anesthetic delivery apparatus 20, and the ventilation module 50. For example, in the embodiment shown in FIG. 3, the gas source module 10 is connected with the anesthetic delivery apparatus 20 through the monitoring apparatus for flow amount 60, wherein the ventilation module 50 is connected with the gas source module 10. In FIG. 3, the gas source interface is connected with the evaporator 210 through the monitoring apparatus for flow amount 60. For example, in the embodiment shown in FIG. 13, the anesthesia system further includes a three-way valve T12. The three-way valve T12 includes a first port 1, which is connected with the monitoring apparatus for flow amount 60, a second port 2, which is connected with the anesthetic delivery apparatus 20, and a third port 3, which is connected with the ventilation module 50. That is, the gas source module 10 is connected with an input end of the monitoring apparatus for flow amount 60. An output end of the monitoring apparatus for flow amount 60 can be connected with the anesthetic delivery apparatus 20 under a control of the three-way valve T12, or connected with the ventilation module 50 under a control of the three-way valve T12. That is, the three-way valve T12 is configured to connect the monitoring apparatus for flow amount 60 to either the anesthetic delivery apparatus 20 or the ventilation module 50.

The gas source module 10 can further include a laughing gas interface for providing a laughing gas. The laughing gas interface is configured to connect an external laughing gas source (such as laughing gas delivery pipelines, laughing gas cylinders in hospitals, etc) or an internal laughing gas source. The monitoring apparatus for flow amount 60 can have three input interfaces, which are respectively connected with the oxygen interface, the air interface, and the laughing gas interface.

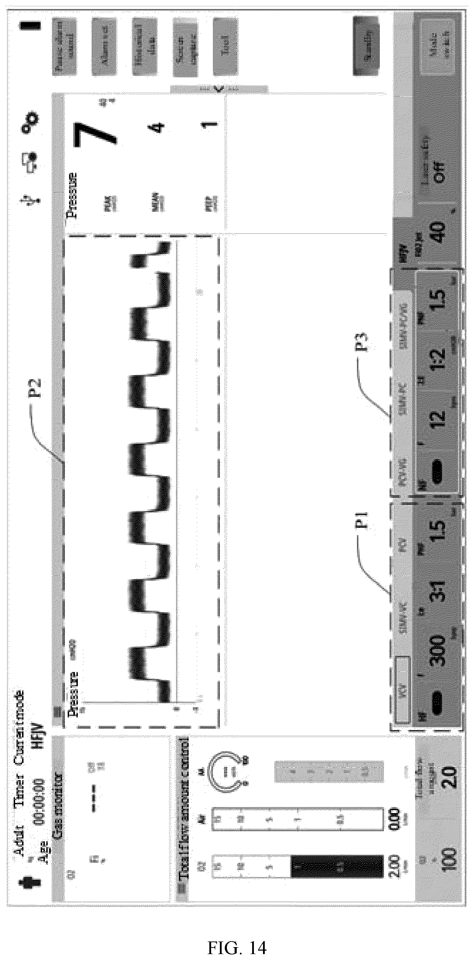

As shown in FIG. 14, when the respiratory support mode, which is received by the human-machine interaction apparatus, is superimposed ventilation, the anesthesia system is configured to display a first display control P1 and a second display control P3 on a display interface of the human-machine interaction apparatus. The anesthesia system receives a user operation on the first display control P1 through the human-machine interaction apparatus, so as to obtain a first ventilation parameter, and receives a user operation on the second display control P3 through the human-machine interaction apparatus, so as to obtain a second ventilation parameter. The first ventilation parameter includes at least one of a first ventilation frequency, a first inspiratory-to-expiratory ratio, and a first driving pressure, while the second ventilation parameter includes at least one of a second ventilation frequency, a second inspiratory-to-expiratory ratio, and a second driving pressure. User can easily set the ventilation frequency, inspiratory-to-expiratory ratio, and the first driving pressure.

The anesthesia system controls the first pressure regulation component and/or the first control component according to the first ventilation parameter, so as to, when providing the respiratory support for the patient, keep a ventilation parameter of the first ventilation apparatus to be consistent with the first ventilation parameter. For example, the user sets the first ventilation frequency through the first display control P1, and the anesthesia system controls the first control component to switch an opening degree of valve according to the first ventilation frequency, during an operation of the first ventilation apparatus. If the user sets the first driving pressure through the first display control, the anesthesia system correspondingly controls the first pressure regulation component according to the first driving pressure, during an operation of the first ventilation apparatus. The anesthesia system controls the second pressure regulation component and/or the second control component according to the second ventilation parameter, so as to, when providing the respiratory support for the patient, keep a ventilation parameter of the second ventilation apparatus to be consistent with the second ventilation parameter. Similarly, if the second ventilation parameter includes the second ventilation frequency, the anesthesia system controls the second control component to switch an opening degree of valve according to the second ventilation frequency, during an operation of the second ventilation apparatus. If the second ventilation parameter includes the second driving pressure, the anesthesia system correspondingly controls the second pressure regulation component according to the second driving pressure, during an operation of the second ventilation apparatus. In this way, user can effectively control the superimposed ventilation of high frequency and normal frequency.

This display interface also provides different respiratory support modes for user to select, making it easy for user to operate.

The anesthesia system is further configured to acquire an airway pressure, and display, on the display interface, of the human-machine interaction apparatus, waveform(s) of the airway pressure. The display interface of the human-machine interaction apparatus further includes a waveform display area P2. The anesthesia system displays waveform(s) of the airway pressure in the waveform display area P2 of the display interface.

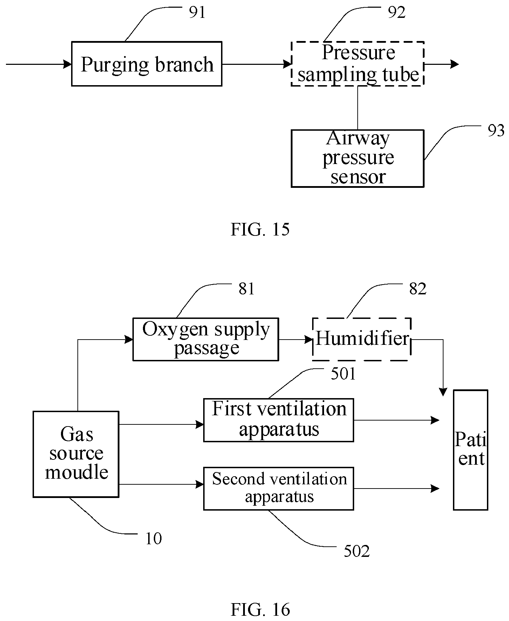

As shown in FIG. 15, the anesthesia system further includes an airway pressure sensor 93 which is configured to acquire an airway pressure. The airway pressure sensor 93 is connected with a pressure sampling tube 92, which is usually arranged at the jet accessory, with its outlet close to an end of the jet accessory. In this way, the pressure near the airway can be conducted to the airway pressure sensor 93 through the pressure sampling tube 92. Due to the special placement of the jet accessory in jet ventilation, in order to avoid a blockage of the pressure sampling tube 92, it is necessary to purge the pressure sampling tube 92 at least during an operation of the ventilation module 50. Therefore, the anesthesia system further includes a purging branch 91.

The purging branch 91 is configured to utilize the gas, which is provided by the gas source module, to output a purged gas to the pressure sampling tube 92, so as to avoid a blockage of the outlet of the pressure sampling tube 92. That is the pressure sampling tube 92 uses a flow of the purged gas to avoid a blockage of the pressure sampling tube 92.

A third control component can also be arranged inside the purging branch 91. When the ventilation module 50 is started, the third control component opens the purging branch 91, so as to provide to the pressure sampling tube 92 the purged gas. Of course, in some embodiments, the purging branch 91 can also automatically or passively start purging, when the gas source module starts supplying gas.

The pressure sampling tube in non-jet ventilation scenarios usually has a larger diameter, and a pressure deviation caused by gas resistance can be ignored, so that the pressure sensor does not need calibration compensation. The pressure sampling tube 92 used in the jet ventilation scenarios usually has a small diameter and high gas resistance. Due to a large pressure deviation caused by the gas resistance, calibration compensation is required. The anesthesia system may further include a control apparatus for calibrating the airway pressure sensor 93 after the purging branch 91 is opened. By calibrating the airway pressure sensor 93, the pressure deviation can be effectively eliminated, and an accuracy of airway pressure detection can be improved.

After the purging branch 91 is opened, the control apparatus can calibrate the airway pressure sensor 93, when the ventilation module does not provide jet ventilation to the patient, thereby avoiding interference caused by ventilation. There are various ways to do this, and two examples are given below for explanation.

The first type is as follows. The control apparatus receives, through the human-machine interaction apparatus, an instruction to start ventilation, which instruction is inputted by a user, in respond to this instruction, waits for a first preset duration and then starts the ventilation module to provide the jet ventilation to the patient; and calibrates airway pressure sensor 93 while waiting for the first preset duration. The first preset duration can be set as needed, for example, the first preset duration can be within 5 seconds, as if it can satisfy a time requirement of the calibration.

The second method is as follows. After the ventilation module 50 provides the jet ventilation to the patient, the control apparatus receives, through the human-machine interaction apparatus, an instruction to detect carbon dioxide, which instruction is inputted by a user, in respond to this instruction, pauses the jet ventilation to the patient by the ventilation module 50, performs a concentration detection of the carbon dioxide and calibrates the airway pressure sensor 93 during a paused period. Specifically, during the paused period, the airway pressure sensor 93 can be calibrated after determining that an airway pressure drop is zero. It can be determined that the airway pressure is decreased to zero by pausing for a second preset duration. The second preset duration can be set as needed, for example, the second preset duration is between 1-6s.

In these two methods, the timing of calibration basically does not affect the ventilation of ventilation module 50, and during calibration, the ventilation module 50 does not output gas, ensuring the accuracy of calibration.

There are multiple ways to calibrate control apparatuses, and two examples are given below.

One method is as follows. The airway pressure sensor 93 is set to zero by the control apparatus during calibration, which means that when the ventilation module is not in operation, but the purging is performed, the airway pressure sensor 93 has a reading which is to be set to zero, regardless of its value. After the ventilation module is turned on, the pressure value detected by the airway pressure sensor 93 is the airway pressure.

Another method is as follows. The control apparatus acquires a current measurement value of the airway pressure sensor 93 during calibration, and uses the current measurement value as a compensation value. When the airway pressure sensor 93 detects the airway pressure, the control apparatus subtracts the compensation value from a measured value of the airway pressure sensor 93 to obtain the airway pressure.

The anesthesia system provided by this disclosure can be applied to an entire machine, that is, the anesthesia system can be an anesthesia machine. In some embodiments, the anesthesia machine may include a main frame, which has a housing, and at least part of the ventilation module may be provided in a containment space formed by the housing, thus forming a collective whole with the anesthesia machine. That is, at least some ventilation modules are integrated inside the anesthesia machine, integrated with the anesthesia machine, and can communicate and connect with the human-machine interaction apparatus of the anesthesia machine.

This disclosure integrates functions of the ventilation module, such as superimposed jet ventilation function, into a general anesthesia machine, and integrates a board card, a display screen, etc., into one device, which brings convenience to the management of operating room device, allows more hospitals to perform anesthesia surgeries, such as pharyngeal, tracheal, bronchial, or lung surgeries, that cannot be performed with tracheal intubation ventilation, and has great clinical value.

This disclosure also provides a respiratory support device, such as an anesthesia machine, a ventilator, etc., which provides respiratory support for patient. As shown in FIGS. 16, the respiratory support device includes the aforementioned gas source module 10, the aforementioned oxygen supply branch, and the aforementioned ventilation module.

The ventilation module includes the first ventilation apparatus 501 and the second ventilation apparatus 502 mentioned above. Similarly, the first ventilation apparatus 501 and the second ventilation apparatus 502 are capable of being controlled to work simultaneously.

The first ventilation apparatus 501 is configured to provide respiratory support for the patient at a first ventilation frequency by using a first gas. The second ventilation apparatus 502 is configured to provide respiratory support for the patient at a first ventilation frequency by using a first gas.

As shown in FIG. 6, the first ventilation apparatus 501 includes a first gas delivery branch 503 and a first control component 505, which is arranged inside the first gas delivery branch 503. The first control component 505 is configured to control an oxygen concentration of the first gas, which is outputted by the first ventilation apparatus 501 through the first gas delivery branch 503.

The second ventilation apparatus 502 includes a second gas delivery branch 506 and a second control component 508 which is arranged inside the second gas delivery branch 506. The second control component 508 is configured to control an oxygen concentration of the first gas, which is outputted by the second ventilation apparatus 502 through the second gas delivery branch 506. The first control component 505 and the second control component 508 are independent control components.

At present, there are jet ventilation ventilators on the market, as shown in FIG. 5. Although they can provide high-frequency jet ventilation and normal-frequency jet ventilation, they do not work simultaneously and do not have a function of superposed jet ventilation. Usually, high-frequency jet ventilation or normal-frequency jet ventilation are selected based on patient condition. From the gas path shown in FIG. 5, if superimposed jet ventilation is wanted, a general solution is as follows. O₂ and air are firstly mixed in an oxygen mixing chamber, that is, the oxygen concentration is controlled. Then the mixed gas is injected into one or two gas volumes, which correspond to high-frequency and normal frequency branches, respectively. The pressure of the gas volume is regulated through a vent valve, that is, the driving pressure is regulated. The gases from two gas volumes are controlled by the vent valve and then jet from the corresponding high-frequency jet port and the normal-frequency jet port. The high-frequency gas flow and normal-frequency gas flow in this scheme are both led out from the oxygen mixing chamber at the front end. It can be seen that there is a problem of mutual interference between the two flows during superimposed ventilation. When the required gas flows for both jet ventilation are large, this interference is more pronounced. At the same time, the pressure regulation method is also achieved through a gas volume. When a large amount of gas in the gas volume is consumed during inhalation, the driving pressure decreased, and it is necessary to supplement the gas in the gas volume to maintain the pressure. This supplement stage is conducted during the exhalation stage. When the exhalation stage is short, sometimes it may not have sufficient time to complete the necessary supplement, which leads to insufficient gas flow during inhalation. In clinical applications, the airway pressure is low, and the ventilation volume is insufficient.

In the embodiment of this disclosure, the first control component 505 and the second control component 508 independently control the oxygen concentration in their respective gas delivery branches, without the need for a mixed oxygen chamber, and never cause problems, such as low airway pressure and insufficient ventilation.

The first gas delivery branch 503 may also be arranged with a first pressure regulation component 504 for controlling a gas pressure, which is provided by the first ventilation apparatus 501. The second gas delivery branch 506 may also be arranged with a second pressure regulation component 507 for controlling a gas pressure, which is provided by the second ventilation apparatus 502. The first pressure regulation component 504 is located upstream of the first control component 505, that is gas first passes through the first pressure regulation component 504 and then passes through the first control component 505; the second pressure regulation component 507 is located upstream of the second control component 508, that is, gas first passes through the second pressure regulation component 507 and then passes through the second control component 508. The first pressure regulation component 504 and the second pressure regulation component 507 are both pressure regulation valves, which control the pressures of gases, which are provided by the first ventilation apparatus 501 and the second ventilation apparatus 502 respectively, by regulating the opening degree of valve. According to the existing technology shown in FIG. 5, the gas in the gas volume during the inspiratory phase decreases and needs to be supplemented in a timely manner. If the inspiratory-to-expiratory ratio is not set properly and there is no time to supplement, the required driving pressure cannot be obtained. The pressure regulation valve used in this disclosure is similar to a proportional valve, which regulates the gas pressure through the opening degree of valve, thus having a fast regulation speed, without requiring gas supplement and deflation.

The gas path structures of the first ventilation apparatus 501 and the second ventilation apparatus 502 can be the same or different. The gas path structures that can be used are detailed in the above embodiments and FIGS. 7-12, and are not elaborated here.

When the respiratory support device is an anesthesia machine, it further includes an anesthesia branch, that is, its structure and function are the same as the above anesthesia system, and are not elaborated here. When the respiratory support device is a respirator, except for the anesthesia branch mentioned above, it can be the same as the anesthesia system mentioned above, so not elaborated here.

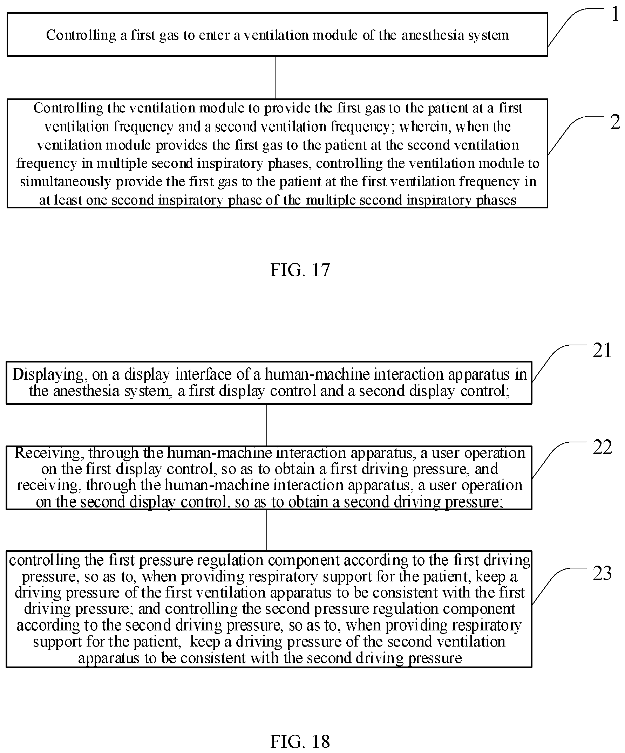

Based on the above anesthesia system, this disclosure also provides a ventilation control method, as shown in FIG. 17, including the following steps.

In step 1, a first gas is controlled to enter into a ventilation module of the anesthesia system. For example, open a valve between the gas source interface in gas source module 10 and the ventilation module.

In step 2, the ventilation module is controlled to provide the first gas to the patient at a first ventilation frequency and a second ventilation frequency. When the ventilation module provides the first gas to the patient at the second ventilation frequency in multiple second inspiratory phases, the ventilation module is controlled to simultaneously provide the first gas to the patient at the first ventilation frequency in at least one second inspiratory phase of the multiple second inspiratory phases. For example, the first ventilation apparatus is controlled to provide respiratory support for the patient at the first ventilation frequency by using the first gas; the second ventilation apparatus is simultaneously configured to provide respiratory support for the patient at the second ventilation frequency by using the first gas. Wherein the first ventilation frequency is higher than the second ventilation frequency. The way in which the ventilation module provides respiratory support for patient has been elaborated in detail in the above embodiments, and is not elaborated here.

As shown in FIG. 18, the method further includes following steps:
step 21, displaying, on a display interface of a human-machine interaction apparatus of the anesthesia system, a first display control and a second display control;
step 22, receiving, through the human-machine interaction apparatus, a user operation on the first display control, so as to obtain a first driving pressure, and receiving, through the human-machine interaction apparatus, a user operation on the second display control, so as to obtain a second driving pressure;
step 23, controlling the first pressure regulation component according to the first driving pressure, so as to, when providing respiratory support for the patient, keep a driving pressure of the first ventilation apparatus to be consistent with the first driving pressure; and controlling the second pressure regulation component according to the second driving pressure, so as to, when providing respiratory support for the patient, keep a driving pressure of the second ventilation apparatus to be consistent with the second driving pressure.

The steps shown in FIG. 18 have been elaborated in detail in the aforementioned embodiments and are not elaborated here.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-arranged with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments are included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. An anesthesia machine, **characterized in that**, comprising an anesthesia branch and a ventilation module;
the anesthesia branch is configured to mix a first gas with an anesthetic, so as to obtain a second gas, and configured to provide the second gas to a patient, so as to provide anesthesia respiratory support for said patient;
the ventilation module is configured to respectively provide the first gas to a patient at a first ventilation frequency and a second ventilation frequency, so as to provide respiratory support for said patient; wherein the ventilation module is further configured to, when providing the first gas to said patient at the second ventilation frequency in multiple second inspiratory phases, simultaneously provide the first gas to said patient at the first ventilation frequency in at least one of the multiple second inspiratory phases.

2. An anesthesia machine, **characterized in that**, comprising an anesthesia branch and a ventilation module;
the anesthesia branch is configured to mix a first gas with an anesthetic, so as to obtain a second gas, and configured to provide the second gas to a patient, so as to provide anesthesia respiratory support for said patient;
the ventilation module is configured to, when a respiratory support mode, which is provided by the anesthesia machine, is superimposed ventilation, simultaneously provide the first gas to a patient at a first ventilation frequency and a second ventilation frequency, so as to provide respiratory support for said patient.

3. The anesthesia machine according to claim 2, **characterized in that**, further comprising a human-machine interaction apparatus, which is configured to receive the respiratory support mode, which is provided by the anesthesia machine; when the respiratory support mode, which is received by the human-machine interaction apparatus, is superimposed ventilation, the anesthesia machine is configured to:
display, on a display interface of the human-machine interaction apparatus, a first display control and a second display control;
receive, through the human-machine interaction apparatus, a user operation on the first display control, so as to obtain a first ventilation parameter; and receive, through the human-machine interaction apparatus, a user operation on the second first display control, so as to obtain a second ventilation parameter; wherein the first ventilation parameter comprises at least one of a first ventilation frequency, a first inspiratory-to-expiratory ratio, and a first driving pressure, the second ventilation parameter comprises at least one of a second ventilation frequency, a second inspiratory-to-expiratory ratio, and a second driving pressure; and
provide respiratory support for said patient according to the first ventilation parameter, and provide respiratory support for said patient according to the second ventilation parameter;
preferably, the anesthesia machine is further configured to acquire an airway pressure, and display, on the display interface, waveform(s) of the airway pressure.

4. The anesthesia machine according to claim 1 or claim 2, **characterized in that**, the ventilation module comprises a first ventilation apparatus and a second ventilation apparatus, which are capable of being controlled to work simultaneously;
the first ventilation apparatus is configured to provide the first gas to said patient at the first ventilation frequency;
the second ventilation apparatus is configured to provide the first gas to said patient at the second ventilation frequency.

5. The anesthesia machine according to claim 4, **characterized in that**, further comprising an oxygen interface, which is connected with an external oxygen source or an internal oxygen source, and/or an air interface, which is connected with an external air source or an internal air source; wherein the first gas comprises oxygen and/or air; the first ventilation apparatus and the second ventilation apparatus are both connected with the oxygen interface and/or the air interface;
wherein anesthesia machine further comprises a fifth pressure regulation valve and a sixth pressure regulation valve, which are both configured to control a gas pressure; wherein the first ventilation apparatus is connected with the oxygen interface through the fifth pressure regulation valve, and/or connected with the air interface through the sixth pressure regulation valve; the second ventilation apparatus is connected with the oxygen interface through the fifth pressure regulation valve, and/or connected with the air interface through the sixth pressure regulation valve.

6. The anesthesia machine according to claim 4, **characterized in that**, further comprising an oxygen interface, which is connected with an external oxygen source or an internal oxygen source, and/or an air interface, which is connected with an external air source or an internal air source; wherein the first gas comprises oxygen and/or air; the first ventilation apparatus and the second ventilation apparatus are both connected with the oxygen interface and/or the air interface;
the first ventilation apparatus comprises a first oxygen branch, a first air branch, and a first output port; an input end of the first oxygen branch is connected with the oxygen interface, and an input end of the first air branch is connected with the air interface; the first oxygen branch and the first air branch are converged with each other and then connected with the first output port; wherein the first oxygen branch is arranged with a first pressure regulation valve, which is configured to control an oxygen pressure inside the first oxygen branch; the first air branch is arranged with a second pressure regulation valve, which is configured to control an air pressure inside the first air branch; the second ventilation apparatus comprises a second oxygen branch, a second air branch, and a second output port; an input end of the second oxygen branch is connected with the oxygen interface, and an input end of the second air branch is connected with the air interface; the second oxygen branch and the second air branch are converged with each other and then connected with the second output port; wherein the second oxygen branch is arranged with a third pressure regulation valve, which is configured to control an oxygen pressure inside the second oxygen branch; the second air branch is arranged with a fourth pressure regulation valve, which is configured to control an air pressure inside the second air branch; or
the anesthesia machine further comprises a fifth pressure regulation valve and a sixth pressure regulation valve, which are both configured to control a gas pressure; the first ventilation apparatus comprises a first oxygen branch, a first air branch, and a first output port; an input end of the first oxygen branch is connected with the oxygen interface through the fifth pressure regulation valve, and an input end of the first air branch is connected with the air interface through the sixth pressure regulation valve; the first oxygen branch and the first air branch are converged with each other and then connected with the first output port; the second ventilation apparatus comprises a second oxygen branch, a second air branch, and a second output port; an input end of the second oxygen branch is connected with the oxygen interface through the fifth pressure regulation valve, and an input end of the second air branch is connected with the air interface through the sixth pressure regulation valve; the second oxygen branch and the second air branch are converged with each other and then connected with the second output port.

7. The anesthesia machine according to claim 6, **characterized in that**:
the first oxygen branch is arranged with a first control valve, which is located downstream of the first pressure regulation valve or the fifth pressure regulation valve; wherein the first control valve is configured to control a flow amount of oxygen inside the first oxygen branch and enable the oxygen inside the first oxygen branch to be provided to said patient at the first ventilation frequency; the first air branch is arranged with a second control valve, which is located downstream of the second pressure regulation valve or the sixth pressure regulation valve; wherein the second control valve is configured to control a flow amount of air inside the first air branch and enable the air inside the first air branch to be provided to said patient at the first ventilation frequency; the second oxygen branch is arranged with a third control valve, which is located downstream of the third pressure regulation valve or the fifth pressure regulation valve; wherein the third control valve is configured to control a flow amount of oxygen inside the second oxygen branch and enable the oxygen inside the second oxygen branch to be provided to said patient at the second ventilation frequency; the second air branch is arranged with a fourth control valve, which is located downstream of the fourth pressure regulation valve or the sixth pressure regulation valve; wherein the fourth control valve is configured to control a flow amount of air inside the second air branch and enable the air inside the second air branch to be provided to said patient at the second ventilation frequency; or
the first ventilation apparatus further comprises a fifth control valve, which is located downstream of a converged node of the first oxygen branch and the first air branch; wherein the fifth control valve is configured to control a flow amount of the first gas which is formed by mixing air and oxygen and enable the first gas to be provided to said patient at the first ventilation frequency; the second ventilation apparatus further comprises a sixth control valve, which is located downstream of a converged node of the second oxygen branch and the second air branch; wherein the sixth control valve is configured to control a flow amount of the first gas which is formed by mixing air and oxygen and enable the first gas to be provided to said patient at the second ventilation frequency.

8. The anesthesia machine according to claim 4, **characterized in that**, further comprising an oxygen interface, which is connected with an external oxygen source or an internal oxygen source, and/or an air interface, which is connected with an external air source or an internal air source; wherein the first gas comprises oxygen and/or air; the first ventilation apparatus and the second ventilation apparatus are both connected with the oxygen interface and/or the air interface;
the first ventilation apparatus comprises a first oxygen branch and a first output port; an input end of the first oxygen branch is connected with the oxygen interface, and an output end of the first oxygen branch is connected with the first output port; wherein the first oxygen branch is arranged with a first pressure regulation valve, which is configured to control an oxygen pressure inside the first oxygen branch; the second ventilation apparatus comprises a second oxygen branch and a second output port; an input end of the second oxygen branch is connected with the oxygen interface, and an output end of the second oxygen branch is connected with the second output port; wherein the second oxygen branch is arranged with a third pressure regulation valve, which is configured to control an oxygen pressure inside the second oxygen branch; or
the anesthesia machine further comprises a fifth pressure regulation valve, which is configured to control a gas pressure; the first ventilation apparatus comprises a first oxygen branch and a first output port; an input end of the first oxygen branch is connected with the oxygen interface through the fifth pressure regulation valve, and an output end of the first oxygen branch is connected with the first output port; the second ventilation apparatus comprises a second oxygen branch and a second output port; an input end of the second oxygen branch is connected with the oxygen interface through the fifth pressure regulation valve, and an output end of the second oxygen branch is connected with the second output port.

9. The anesthesia machine according to claim 8, **characterized in that**:
the first oxygen branch is arranged with a first control valve, which is located downstream of the first pressure regulation valve or the fifth pressure regulation valve; wherein the first control valve is configured to control a flow amount of oxygen inside the first oxygen branch and enable the oxygen inside the first oxygen branch to be provided to said patient at the first ventilation frequency; and
the second oxygen branch is arranged with a third control valve, which is located downstream of the third pressure regulation valve or the fifth pressure regulation valve; wherein the third control valve is configured to control a flow amount of oxygen inside the second oxygen branch and enable the oxygen inside the second oxygen branch to be provided to said patient at the second ventilation frequency.

10. The anesthesia machine according to claim 4, **characterized in that**, further comprising an oxygen interface, which is connected with an external oxygen source or an internal oxygen source, and/or an air interface, which is connected with an external air source or an internal air source; wherein the first gas comprises oxygen and/or air; the first ventilation apparatus and the second ventilation apparatus are both connected with the oxygen interface and/or the air interface;
the first ventilation apparatus comprises a first air branch and a first output port; an input end of the first air branch is connected with the air interface, and an output end of the first air branch is connected with the first output port; wherein the first air branch is arranged with a second pressure regulation valve, which is configured to control an air pressure inside the first air branch; the second ventilation apparatus comprises a second air branch and a second output port; an input end of the second air branch is connected with the air interface, and an output end of the second air branch is connected with the second output port; wherein the second air branch is arranged with a fourth pressure regulation valve, which is configured to control an air pressure inside the second air branch; or
the anesthesia machine further comprises a sixth pressure regulation valve, which is configured to control a gas pressure; the first ventilation apparatus comprises a first air branch and a first output port; an input end of the first air branch is connected with the air interface through the sixth pressure regulation valve, and an output end of the first air branch is connected with the first output port; the second ventilation apparatus comprises a second air branch and a second output port; an input end of the second air branch is connected with the air interface through the sixth pressure regulation valve, and an output end of the second air branch is connected with the second output port.

11. The anesthesia machine according to claim 10, **characterized in that**:
the first air branch is arranged with a second control valve, which is located downstream of the second pressure regulation valve or the sixth pressure regulation valve; wherein the second control valve is configured to control a flow amount of air inside the first air branch and enable the air inside the first air branch to be provided to said patient at the first ventilation frequency;
the second air branch is arranged with a fourth control valve, which is located downstream of the fourth pressure regulation valve or the sixth pressure regulation valve; wherein the fourth control valve is configured to control a flow amount of air inside the second air branch and enable the air inside the second air branch to be provided to said patient at the second ventilation frequency.

12. The anesthesia machine according to claim 1 or 2, **characterized in that**, a driving pressure of the first ventilation apparatus for providing respiratory support for said patient, is lower than a driving pressure of the second ventilation apparatus for providing respiratory support for said patient; or
the first ventilation frequency is not lower than 50bpm, and the second ventilation frequency is lower than 100bpm; or
in order to provide respiratory support for said patient, the anesthesia machine is further configured to provide different flow amounts of the first gas to said patient in two different time periods of a respiratory cycle; wherein a flow amount in one time period is lower than a flow amount in the other time period, or a flow amount in one time period is zero; or
the anesthesia machine further comprises a purging branch and an airway pressure sensor, wherein the airway pressure sensor is connected with a pressure sampling tube; the purging branch is configured to provide a purging gas to the pressure sampling tube, and is arranged with a third control component; wherein the third control component is configured to start the purging branch, so as to enable the purging branch to provide the purging gas to the pressure sampling tube, when the ventilation module is started.

13. The anesthesia machine according to claim 4, **characterized in that**:
the first ventilation apparatus comprises a first control component which is configured to control an oxygen concentration of the first gas, which is outputted by the first ventilation apparatus; the second ventilation apparatus comprises a second control component which is configured to control an oxygen concentration of the first gas, which is outputted by the second ventilation apparatus; wherein the first control component and the second control component are independent control components;
preferably, the anesthesia machine further comprises first pressure regulation component, which is located upstream of the first control component, and a second pressure regulation component, which is located upstream of the second control component; wherein the first pressure regulation component and the second pressure regulation component are pressure regulation valves, which are configured to respectively regulate, through regulating opening degrees of the pressure regulation valves, a gas pressure which is provided by the first ventilation apparatus and a gas pressure which is provided by the second ventilation apparatus .

14. The anesthesia machine according to claim 1 or claim 2, **characterized in that**, the anesthesia branch comprises a monitoring apparatus for flow amount, an anesthetic delivery apparatus which is connected with the monitoring apparatus for flow amount, a respiratory loop, and a ventilation control apparatus; wherein:
the monitoring apparatus for flow amount is configured to regulate a flow amount of the first gas;
the anesthetic delivery apparatus is configured to mix the first gas with the anesthetic, so as to obtain the second gas, and configured to provide the second gas to the respiratory loop;
the respiratory loop is configured to provide the second gas to said patient; and
the ventilation control apparatus is configured to control the respiratory loop to provide the second gas to said patient, so as to enable the anesthesia machine to provide anesthesia respiratory support for said patient; and/or
the anesthesia machine further comprises a housing, wherein at least part of the anesthesia branch and at least part of the ventilation module are arranged inside a containment space, which is formed by the housing.

15. A respiratory support device, **characterized in that**, comprising a first ventilation apparatus and a second ventilation apparatus, which are capable of being controlled to work simultaneously, so as to provide respiratory support for a patient;
the first ventilation apparatus is configured to provide a first gas to the patient at a first ventilation frequency, and the second ventilation apparatus is configured to provide a first gas to the patient at a second ventilation frequency;
the first ventilation apparatus comprises a first gas delivery branch and a first control component which is arranged inside the first gas delivery branch, wherein the first control component is configured to control an oxygen concentration of the first gas, which is outputted by the first ventilation apparatus through the first gas delivery branch; the second ventilation apparatus comprises a second gas delivery branch and a second control component which is arranged inside the second gas delivery branch, wherein the second control component is configured to control an oxygen concentration of the first gas, which is outputted by the second ventilation apparatus through the second gas delivery branch; wherein the first control component and the second control component are independent control components.
